(19) Europäisches Patentamt
European Patent Office
Office européen des brevets

(11) EP 2 025 348 A1

(12) # EUROPEAN PATENT APPLICATION

(43) Date of publication:
**18.02.2009 Bulletin 2009/08**

(51) Int Cl.:
***A61K 47/48*** (2006.01) ***A61P 35/00*** (2006.01)

(21) Application number: **07015900.9**

(22) Date of filing: **13.08.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
- **Max-Planck-Gesellschaft zur Förderung der Wissenschaften e.V.
80539 München (DE)**
- **Johannes- Gutenberg-Universität Mainz
55122 Mainz (DE)**

(72) Inventors:
- **Müllen, Klaus
50939 Köln (DE)**
- **Alemdaroglu, Fikri E.
49448 Stemshorn (DE)**
- **Herrmann, Andreas
65199 Wiesbaden (DE)**
- **Alemdaroglu, N. Ceren
49448 Stemshorn (DE)**
- **Langguth, Peter
63599 Biebergemünd (DE)**

(74) Representative: **Vossius & Partner
Siebertstrasse 4
81675 München (DE)**

(54) **Targeted block copolymer micelles**

(57) The present invention provides a micelle comprising an amphiphilic block copolymer, said amphiphilic block copolymer consisting of (a) a hydrophobic polymer attached to the 5' end of a first nucleic acid molecule, wherein said first nucleic acid molecule is hybridized with a second nucleic acid molecule, wherein a targeting unit capable of selectively binding to a specific cell type and/or tissue is attached to the 5' end of said second nucleic acid molecule; and/or (b) a hydrophobic polymer attached to the 3' end of a first nucleic acid molecule, wherein said first nucleic acid molecule is hybridized with a second nucleic acid molecule, wherein a targeting unit capable of selectively binding to a specific cell type and/or tissue is attached to the 3' end of said second nucleic acid molecule.

EP 2 025 348 A1

SEQUENCE LISTING

<110> Max-Planck Gesellschaft zur Förderung der Wissenschaften e.V.

<120> Targeted Block Copolymer Micelles

<130> N1872 EP

<160> 46

<170> PatentIn version 3.4

<210> 1
<211> 20
<212> RNA
<213> artificial sequence

<220>
<221> source
<223> /note="Description of artificial sequence:immunostimulating sequence"

<400> 1
ggacugcguu cgcgcuuucc 20

<210> 2
<211> 22
<212> RNA
<213> artificial sequence

<220>
<221> source
<223> /note="Description of artificial sequence:immunostimulating sequence"

<400> 2
ggcuuaucca uugcacuccg ga 22

<210> 3
<211> 16
<212> RNA
<213> artificial sequence

<220>
<221> source
<223> /note="Description of artificial sequence:immunostimulating sequence"

<400> 3
gacuagcuug cuguuu 16

<210> 4
<211> 20
<212> RNA
<213> artificial sequence

<220>
<221> source
<223> /note="Description of artificial sequence:immunostimulating sequence"

<400> 4
uuugugguag ugggggacug 20

<210> 5
<211> 19
<212> RNA

<213> artificial sequence

<220>
<221> source
<223> /note="Description of artificial sequence:immunostimulating sequence"

<400> 5
acgaaggugg uuuucccag 19

<210> 6
<211> 20
<212> RNA
<213> artificial sequence

<220>
<221> source
<223> /note="Description of artificial sequence:immunostimulating sequence"

<400> 6
aaacaacaaa cacacaaacc 20

<210> 7
<211> 15
<212> RNA
<213> artificial sequence

<220>
<221> source
<223> /note="Description of artificial sequence:immunostimulating sequence"

<400> 7
accuggcagg ggaga 15

<210> 8
<211> 13
<212> RNA
<213> artificial sequence

<220>
<221> source
<223> /note="Description of artificial sequence:immunostimulating sequence"

<400> 8
cccagggcga ggc 13

<210> 9
<211> 20
<212> RNA
<213> artificial sequence

<220>
<221> source
<223> /note="Description of artificial sequence:immunostimulating sequence"

<400> 9
ggacugcguu guggcuuucc 20

<210> 10
<211> 12
<212> RNA
<213> artificial sequence

<220>

<221> source
<223> /note="Description of artificial sequence:immunostimulating sequence"

<400> 10
gauacuuacc ug 12

<210> 11
<211> 6
<212> RNA
<213> artificial sequence

<220>
<221> source
<223> /note="Description of artificial sequence:immunostimulating sequence"

<400> 11
gauacu 6

<210> 12
<211> 9
<212> RNA
<213> artificial sequence

<220>
<221> source
<223> /note="Description of artificial sequence:immunostimulating sequence"

<400> 12
aauuuuuga 9

<210> 13
<211> 9
<212> RNA
<213> artificial sequence

<220>
<221> source
<223> /note="Description of artificial sequence:immunostimulating sequence"

<400> 13
aacccccga 9

<210> 14
<211> 9
<212> RNA
<213> artificial sequence

<220>
<221> source
<223> /note="Description of artificial sequence:immunostimulating sequence"

<400> 14
aauuugugg 9

<210> 15
<211> 9
<212> RNA
<213> artificial sequence

<220>
<221> source
<223> /note="Description of artificial sequence:immunostimulating sequence"

<400> 15
aacccgcgg 9

<210> 16
<211> 20
<212> RNA
<213> artificial sequence

<220>
<221> source
<223> /note="Description of artificial sequence:immunostimulating sequence"

<400> 16
guaguguuug ugggggacug 20

<210> 17
<211> 20
<212> RNA
<213> artificial sequence

<220>
<221> source
<223> /note="Description of artificial sequence:immunostimulating sequence"

<400> 17
guaguggggg acuguuugug 20

<210> 18
<211> 11
<212> RNA
<213> artificial sequence

<220>
<221> source
<223> /note="Description of artificial sequence:immunostimulating sequence"

<400> 18
gacuagccuu u 11

<210> 19
<211> 20
<212> RNA
<213> artificial sequence

<220>
<221> source
<223> /note="Description of artificial sequence:immunostimulating sequence"

<400> 19
gcccgucugu ugugugacuc 20

<210> 20
<211> 20
<212> RNA
<213> artificial sequence

<220>
<221> source
<223> /note="Description of artificial sequence:immunostimulating sequence"

<400> 20
gcccgacaga agagagacac 20

<210> 21
<211> 20
<212> RNA
<213> artificial sequence

<220>
<221> source
<223> /note="Description of artificial sequence:immunostimulating sequence"

<400> 21
acccaucuau uauauaacuc 20

<210> 22
<211> 20
<212> DNA
<213> artificial sequence

<220>
<221> source
<223> /note="Description of artificial sequence:immunostimulating sequence"

<400> 22
tccatgacgt tcctgatgct 20

<210> 23
<211> 20
<212> DNA
<213> artificial sequence

<220>
<221> source
<223> /note="Description of artificial sequence:immunostimulating sequence"

<400> 23
tccatgacgt tcctgacgtt 20

<210> 24
<211> 24
<212> DNA
<213> artificial sequence

<220>
<221> source
<223> /note="Description of artificial sequence:immunostimulating sequence"

<400> 24
tcgtcgtttt gtcgttttgt cgtt 24

<210> 25
<211> 15
<212> DNA
<213> artificial sequence

<220>
<221> source
<223> /note="Description of artificial sequence:immunostimulating sequence"

<400> 25
tcctggcggg gaagt 15

<210> 26
<211> 19

<212> DNA
<213> artificial sequence

<220>
<221> source
<223> /note="Description of artificial sequence:immunostimulating sequence"

<400> 26
gggggacgat cgtcgggggg 19

<210> 27
<211> 22
<212> DNA
<213> artificial sequence

<220>
<221> source
<223> /note="Description of artificial sequence:immunostimulating sequence"

<400> 27
tcgtcgtttt cggcgcgcgc cg 22

<210> 28
<211> 22
<212> DNA
<213> artificial sequence

<220>
<221> source
<223> /note="Description of artificial sequence:Example 1, first nucleic acid"

<400> 28
cctcgctctg ctaatcctgt ta 22

<210> 29
<211> 22
<212> DNA
<213> artificial sequence

<220>
<221> source
<223> /note="Description of artificial sequence:Example 1, second nucleic acid"

<400> 29
taacaggatt agcagagcga gg 22

<210> 30
<211> 24
<212> DNA
<213> artificial sequence

<220>
<221> source
<223> /note="Description of artificial sequence:Primer"

<400> 30
tttcagattg catcttctct gtct 24

<210> 31
<211> 24
<212> DNA
<213> artificial sequence

<220>
<221> source
<223> /note="Description of artificial sequence:Primer"

<400> 31
gaagtagatg atggacagga tcag 24

<210> 32
<211> 24
<212> DNA
<213> artificial sequence

<220>
<221> source
<223> /note="Description of artificial sequence:Primer"

<400> 32
ttctagtgtg ggtggctgta gtag 24

<210> 33
<211> 24
<212> DNA
<213> artificial sequence

<220>
<221> source
<223> /note="Description of artificial sequence:Primer"

<400> 33
cacagtggtt ccagttgaat ctat 24

<210> 34
<211> 23
<212> DNA
<213> artificial sequence

<220>
<221> source
<223> /note="Description of artificial sequence:Primer"

<400> 34
cttatgcaaa gaggactgtc agc 23

<210> 35
<211> 24
<212> DNA
<213> artificial sequence

<220>
<221> source
<223> /note="Description of artificial sequence:Primer"

<400> 35
ctgaccttgt atgagtgact ccag 24

<210> 36
<211> 27
<212> DNA
<213> artificial sequence

<220>
<221> source
<223> /note="Description of artificial sequence:Probe"

<400> 36
tccgcaagca gttccagtta tactccg 27

<210> 37
<211> 27
<212> DNA
<213> artificial sequence

<220>
<221> source
<223> /note="Description of artificial sequence:Probe"

<400> 37
catttctact tccccacacc cactgtt 27

<210> 38
<211> 27
<212> DNA
<213> artificial sequence

<220>
<221> source
<223> /note="Description of artificial sequence:Probe"

<400> 38
ttgttaactc ctgaggtcca gtcccat 27

<210> 39
<211> 23
<212> DNA
<213> artificial sequence

<220>
<221> source
<223> /note="Description of artificial sequence:Primer"

<400> 39
accatcatca ctttcattgt ctc 23

<210> 40
<211> 22
<212> DNA
<213> artificial sequence

<220>
<221> source
<223> /note="Description of artificial sequence:Primer"

<400> 40
atggacagga tcaggaagta ca 22

<210> 41
<211> 23
<212> DNA
<213> artificial sequence

<220>
<221> source
<223> /note="Description of artificial sequence:Primer"

<400> 41
actggacttc agggtttaac aag 23

<210> 42
<211> 24
<212> DNA
<213> artificial sequence

<220>
<221> source
<223> /note="Description of artificial sequence:Primer"

<400> 42
gtaggagtga gtccagattt catt 24

<210> 43
<211> 21
<212> DNA
<213> artificial sequence

<220>
<221> source
<223> /note="Description of artificial sequence:Primer"

<400> 43
tatgcaaaga ggactgtcag c 21

<210> 44
<211> 21
<212> DNA
<213> artificial sequence

<220>
<221> source
<223> /note="Description of artificial sequence:Primer"

<400> 44
gggaagtagg actcaaaggt g 21

<210> 45
<211> 22
<212> DNA
<213> artificial sequence

<220>
<221> source
<223> /note="Description of artificial sequence:Primer"

<400> 45
agcctcaaga tcatcagcaa tg 22

<210> 46
<211> 23
<212> DNA
<213> artificial sequence

<220>
<221> source
<223> /note="Description of artificial sequence:Primer"

<400> 46
cacgatacca aagttgtcat gga 23

## Description

The present invention relates to a micelle comprising an amphiphilic block copolymer, said amphiphilic block copolymer consisting of (a) a hydrophobic polymer attached to the 5' end of a first nucleic acid molecule, wherein said first nucleic acid molecule is hybridized with a second nucleic acid molecule, wherein a targeting unit capable of selectively binding to a specific cell type and/or tissue is attached to the 5' end of said second nucleic acid molecule; and/or (b) a hydrophobic polymer attached to the 3' end of a first nucleic acid molecule, wherein said first nucleic acid molecule is hybridized with a second nucleic acid molecule, wherein a targeting unit capable of selectively binding to a specific cell type and/or tissue is attached to the 3' end of said second nucleic acid molecule.

A variety of documents is cited throughout this specification. The disclosure content of said documents including manufacturer's manuals is herewith incorporated by reference in its entirety.

Selective drug targeting of a specific organ or tissue is a challenging task. This holds especially true for chemotherapeutic cancer treatment because most of the available anticancer agents cannot distinguish between cancerous and healthy cells, leading to systemic toxicity and undesirable side effects. One approach to address this problem is the use of targeting units such as folate for tumour-specific delivery (Leamon, CP and Reddy, JA; Adv. Drug Delivery Rev. 2004; 56:1127). Drug delivery systems as for instance dendrimers, highly branched macromolecules, can be equipped with folate and an anticancer drug (Quintana, A. et al.; Pharm Res 2002; 19:1310). Different polymeric systems such as shell crosslinked nanoparticles (SCKs) (Pan, D. et al.; Chem. Commun. 2003; 2400), poly(D,L-lactic-co-glycolic acid)-b-poly(ethylene glycol) (Yoo, HS and Park, TG; J. Controlled Release 2004; 96:273) or poly(ethylene glycol-b-ε-caprolactone) (Park, EK et al.; J. Controlled Release 2005; 109:158) block copolymers as well as poly(N-isopropylacrylamide acrylic acid) (Das, M et al.; Adv. Mater. 2006; 18:80) microgels have also been utilized in combination with targeting units. However, conjugates of these polymers with targeting units or drugs are generally ill-defined in structural terms. Furthermore, conjugating two or more different molecules to a given polymer is synthetically challenging, time-consuming and cost-ineffective.

Recently, a new type of amphiphilic block copolymer has emerged that comprises a hydrophobic synthetic polymer component and a biological segment consisting of an oligodeoxynucleotide (ODN) sequence (Alemdaroglu, FE and Herrmann, A; Org. Biomol. Chem. 2007; DOI:10.1039/b61794j; Alemdaroglu, FE et al.; Chem. Commun. 2007; 1358; Ding, K et al.; Angew. Chem., Int. Ed. 2007; 46:1172). Micelles composed of these materials exhibit a corona of single-stranded (ss) DNA and have been utilized for the delivery of anti-sense oligodeoxynucleotides (Jeong, JH and Park, TG; Bioconjugate Chem. 2001; 12:917), for the hybridization with DNA-coated gold nanoparticles (Li, Z et al.; Nano Lett 2004, 4:1055) as well as programmable, three-dimensional scaffolds for DNA-templated organic reactions (Alemdaroglu, FE et al.; Angew. Chem., Int. Ed. 2006; 45:4206).

The technical problem underlying the present invention was to provide alternative and/or improved means and methods for drug delivery.

The solution to this technical problem is achieved by the embodiments characterized in the claims.

Accordingly, the present invention relates in a first embodiment to a micelle comprising an amphiphilic block copolymer, said amphiphilic block copolymer consisting of (a) a hydrophobic polymer attached to the 5' end of a first nucleic acid molecule, wherein said first nucleic acid molecule is hybridized with a second nucleic acid molecule, wherein a targeting unit capable of selectively binding to a specific cell type and/or tissue is attached to the 5' end of said second nucleic acid molecule; and/or (b) a hydrophobic polymer attached to the 3' end of a first nucleic acid molecule, wherein said first nucleic acid molecule is hybridized with a second nucleic acid molecule, wherein a targeting unit capable of selectively binding to a specific cell type and/or tissue is attached to the 3' end of said second nucleic acid molecule.

A micelle according to the invention is an aggregate of amphiphilic molecules presenting a hydrophilic corona formed by the hydrophilic regions of said amphiphilic molecules and sequestering the hydrophobic regions of said amphiphilic molecules in the interior of the micelle. Micelles according to the invention are three-dimensional entities and preferably substantially spherical in shape. The shape and size of the micelle is a function of the molecular geometry of its constituent molecules and solution conditions such as surfactant concentration, temperature, pH, and ionic strength. Generally, micelles are formed when the concentration of the constituent amphiphilic molecules in a hydrophilic solution, preferably aqueous solution, exceeds a certain value, wherein said value is referred to as critical micelle concentration (CMC) which may be determined by using a fluorescent probe, such as pyrene, which partitions into the hydrophobic core of the micelles formed above the CMC value. More specifically, micelles according to the invention form by self-aggregation of amphiphilic block copolymers in hydrophilic, preferably aqueous solutions. Upon formation of the micelles, the hydrophilic regions of said amphiphilic block copolymers are in contact with the surrounding solvent, whereas the hydrophobic regions are buried in the centre of the micelle. Without being bound by a specific theory, the hydrophobic effect, i.e., the tendency of hydrophobic moieties to avoid contact with water, is considered as a driving force behind the formation of micelles. The centre of a micelle according to the invention may comprise further substances, in particular substances with a preference for hydrophobic environments, such as hydrophobic pharmaceutically active agents the delivery of which to biological systems is desired. The micelle according to the invention may also be referred to as a "polymeric nanoparticle" because of its size in the nanometer range and its constituents being of polymeric nature.

The indefinite article "a" or "an" is understood to have the meaning of "one or more". In particular with regard to the amphiphilic block copolymers according to the invention, it is envisaged that one micelle according to the invention comprises a plurality of said amphiphilic block copolymers as defined in the main embodiment.

Preferably, the majority of the constituent amphiphilic moieties are amphiphilic block copolymers as defined in the main embodiment. In other words, micelles according to the invention comprising 50, 60, 70, 80, 90, 95, 98 or 99 weight percent amphiphilic block copolymers as defined in the main embodiment are deliberately envisaged. In another preferred embodiment, a micelle according to the invention consists of amphiphilic block copolymers as defined in the main embodiment.

The term "copolymer" refers to polymers comprising two or more distinct types of monomers. The term "polymer" is understood to comprise both polymers in the narrow sense, i.e. molecules formed from a plurality of monomers, wherein upon formation of the polymer from the monomers no further molecules such as water is formed, as well as polycondensates, i.e., polymers according to the present invention, wherein upon formation of the polymer from the monomers further molecules such as water are formed in addition to the polymer.

Preferred copolymers according to the invention are copolymers formed from two distinct types of monomers. A "type of monomer" refers to a class of molecules exhibiting the same functional groups for the formation of inter-monomer bonds within the polymer. A preferred type of monomer is a nucleotide. Another preferred type of monomers are $\alpha$-hydroxy acids.

The term "block copolymer" refers to copolymers wherein monomers of a given type are organized in blocks, i.e. monomers of the same type are adjacent to each other as opposed to a, for example, alternating sequence of monomers of different types. To explain further, the term "block copolymer" includes molecules of the type $A_iB_jA_kP_l$, wherein A and B designate distinct types, of monomers and the indices i, j, k and l are integer numbers greater than 1. Preferred block copolymers according to the invention are di-block copolymers of the general formula $A_iB_j$. However, also other block copolymers are envisaged, provided they are amphiphilic.

The term "amphiphilic block copolymer" according to the invention designates block copolymers, comprising or consisting of a hydrophobic part and a hydrophilic part, wherein either or both parts may be made of one or more type of momomers, the monomers being organised in blocks. Preferably, the term "amphiphilic block copolymer" relates to di-block copolymers of the general formula $A_iB_j$, wherein one of $A_i$ or $B_j$ is a hydrophobic polymer and the respective other moiety is a hydrophilic polymer.

The amphiphilic block copolymer which is an essential constituent of the micelle according to the invention is a copolymer comprising a hydrophobic polymer attached to a nucleic acid molecule. Upon self-aggregation of these "amphiphilic block copolymers" in aqueous solutions the hydrophobic polymer is located in the centre of the micelle while the nucleic acid moiety is pointing to the surface of the micelle. In case (a) according to the main embodiment the hydrophobic polymer is attached to the 5' end of the nucleic acid molecule. In case (b) the hydrophobic polymer is attached to the 3' end of the nucleic acid molecule. Methods to attach hydrophobic polymers to the 5' end or 3' end, respectively, of nucleic acid molecules are known to the skilled person and include, but are not limited to, for example, automated solid phase synthesis (see, e.g., Alemdaroglu, FE et al.; Angew. Chem., Int. Ed. 2006; 45:4206). To explain further, a hydroxyl end-functionalized hydrophobic polymer may be phosphitylated to obtain the corresponding phosphoramidite terminated polymer. This polymer in turn can be attached to the detrytilated OH-5'-end of the growing oligonucleotide on a solid support, for example in the DNA synthesizer. As familiar to the skilled person, an end-functionalized oligonucleotide and an appropriate end-functionalized hydrophobic polymer can be coupled also by different coupling strategies in solution, wherein suitable coupling strategies include amide bond formation, Michael addition, and Huisgen cycloaddition.

The terms "hydrophilic" and "hydrophobic" have a well recognised meaning in the art. "Hydrophilic" designates a preference for aqueous environments, whereas "hydrophobic" designates a preference for apolar environments. These terms may be functionally defined by reference to amphiphilic block copolymers and micelles according to the invention. A block copolymer consisting of a hydrophobic and a hydrophilic part forms a micelle under physiological conditions. Physiological conditions in accordance with the present invention may vary significantly, for example when comparing the interior of a cell to the extracellular space. Exemplary intracellular conditions comprise 14 mM $Na^+$, 140 mM $K^+$, $10^{-7}$ mM $Ca^{2+}$, 20 mM $Mg^{2+}$, 4 mM $Cl^-$, 10 mM $HCO_3^-$ , 11 mM $HPO_4^{2-}$ and $H_2PO_4^-$ , 1 mM $SO_4^{2-}$, 45 mM phosphocreatine, 14 mM carnosine, 8 mM amino acids, 9 mM creatine, 1.5 mM lactate, 5 mM ATP, 3.7 mM hexose monophosphate, 4 mM protein and 4 mM urea. Exemplary interstitial conditions comprise 140 mM $Na^+$, 4 mM $K^+$, 1.2 mM $Ca^{2+}$, 0.7 mM $Mg^{2+}$, 108 mM $Cl^-$, 28.3 mM $HCO_3^-$, 2 mM $HPO_4^{2-}$ and $H_2PO_4^-$, 0.5 mM $SO_4^{2-}$, 2 mM amino acids, 0.2 mM creatine, 1.2 mM lactate, 5.6 mM glucose, 0.2 mM protein and 4 mM urea. Alternatively, the formation of micelles may be assessed in phosphate buffered saline (PBS), which is a commonly used buffer solution. A 10 liter stock of 10x PBS can be prepared by dissolving 800 g NaCl, 20 g KCl, 144 g $Na_2HPO_4$ and 24 g $KH_2PO_4$ in 8 L of distilled water, and topping up to 10 L. The pH is ~6.8, but when diluted to 1x PBS it changes to 7.4. On dilution, the resultant 1x PBS will have a final concentration of 137 mM NaCl, 10 mM Phosphate, 2.7 mM KCl, pH 7.4.

Alternatively, the logP value may be used for determining whether a molecule is hydrophobic or hydrophilic. Hydrophobicity is also commonly referred to as lipophilicity. The mass flux of a molecule at the interface of two immiscible or substantially immiscible solvents is governed by its lipophilicity. The more lipophilic a molecule is, the more soluble it is in the lipophilic organic phase. The partition coefficient of a molecule that is observed between water and n-octanol has been adopted as the standard measure of lipophilicity. The partition coefficient P of a species A is defined as the ratio $P = [A]_{n\text{-octanol}} / [A]_{water}$. A figure commonly reported is the logP value, which is the logarithm of the partition coefficient. In case a molecule is ionizable, a plurality of distinct microspecies (ionized and not ionized forms of the molecule) will in principle be present in both phases. The quantity describing the overall lipophilicity of an ionizable species is the distribution coefficient D, defined as the ratio D = [sum of the concentrations of all microspecies]$_{n\text{-octanol}}$ /[sum of the concentrations of all microspecies] water. Analogous to logP, frequently the logarithm of the distribution coefficient, logD, is reported.

If the lipophilic character of a substituent on a first molecule is to be assessed and/or to be determined quantitatively, one may assess a second molecule corresponding to that substituent, wherein said second molecule is obtained, for example, by breaking the bond connecting said substituent to the remainder of the first molecule and connecting (the) free valence(s) obtained thereby to hydrogen(s).

Alternatively, the contribution of the substituent to the logP of a molecule may be determined. The contribution $\pi_X$ of a substituent X to the logP of a molecule R-X is defined as $\pi_X = \log P_{R\text{-}X} - \log P_{R\text{-}H}$, wherein R-H is the unsubstituted parent compound. Values of P and D greater than one as well as logP, logD and $\pi_X$ values greater than zero indicate lipophilic/hydrophobic character, whereas values of P and D smaller than one as well as logP, logD and $\pi_X$ values smaller than zero indicate hydrophilic character of the respective molecules or substituents.

Preferably said hydrophobic polymer has a molecular weight between 1000 and 15000, more preferred between 5000 and 10000 Daltons.

"Nucleic acid molecules" in accordance with the present invention include any type of nucleic acid such as DNA, including cDNA or genomic DNA, and RNA. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey; Chem Biol 2001; 8:1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art. Further included are peptide nucleic acids (PNAs).

For the purposes of the present invention, a peptide nucleic acid (PNA) is a polyamide type of nucleic acid analog. The monomeric units for the corresponding derivatives of adenine, guanine, thymine and cytosine are available commercially (for example from Perceptive Biosystems). PNA is a synthetic nucleic acid-mimic with an amide backbone in place of the sugar-phosphate backbone of DNA or RNA. As a consequence, certain components of DNA or RNA, such as phosphorus, phosphorus oxides, or (deoxy)ribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. Furthermore, they are stable under acidic conditions and resistant to proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060). In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point ($T_m$) by 8°-20° C, vs. 4°-16° C for the DNA/DNA 15-mer duplex. Thereby discrimination between perfect matches and mismatches is improved. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to probe molecules. The exact conditions which determine the stringency of hybridization depend on factors such as length of nucleic acids, base composition, percent and distribution of mismatch between the hybridizing sequences, temperature, ionic strength, concentration of destabilizing agents, chemical structure and molecular weight of the hydrophobic polymer and other factors (see also further below). Thus, high stringency conditions can be determined empirically. In one embodiment, the hybridization conditions for specific hybridization are moderate stringency. In a particularly preferred embodiment, the hybridization conditions for specific hybridization are high stringency. High stringency conditions may also be defined as conditions which allow distinguishing between full complementarity over a length of the hybrid of at least 8 base pairs on the one hand and one or more mismatches within a length of the hybrid of at least 8 base pairs on the other hand. Under high stringency conditions a hybrid exhibiting full complementarity over a length of the hybrid of at least 8 base pairs remains stable whereas a hybrid with one or more mismatches within a length of the hybrid of at least 8 base pairs dissociates.

Preferably, the nucleic acid molecules according to the invention are oligonucleotides. It is furthermore preferred that the nucleic acid molecules are single-stranded. The term "oligonucleotide" according to the invention designates nucleic acids of less than about 40 nucleotides or monomers in length. At the same time it is noted that also longer nucleic acids are deliberately envisaged, such as polynucleotides having a length of about 50, 60, 70, 80, 90, 100, 150 or 200 nucleotides or monomers.

In a further preferred embodiment of the invention, the hybrid formed by said first nucleic and said second nucleic acid is a small interfering RNA (siRNA).

The term "small interfering RNA" (siRNA), sometimes known as short interfering RNA or silencing RNA, refers to a class of generally short and double-stranded RNA molecules that play a variety of roles in biology and, to an increasing extent, in treatment of a variety of diseases and conditions. Most notably, siRNA is involved in the RNA interference (RNAi) pathway where the siRNA interferes with the expression of a specific gene (see, e.g. Zamore Nat Struct Biol 2001, 8(9):746-50; Tuschl T. CHEMBIOCHEM. 2001, 2:239-245; Scherr and Eder, Cell Cycle. 2007 Feb;6 (4):444-9; Leung and Whittaker, Pharmacol Ther. 2005 Aug;107(2):222-39; de Fougerolles et al., Nat. Rev. Drug Discov. 2007, 6: 443-453).

Such siRNAs are generally 18-27 nt long, generally comprising a short (usually 19-21-nt) double-strand of RNA (dsRNA) with or without 2-nt 3' overhangs on either end. Each strand can have a 5' phosphate group and a 3' hydroxyl (-OH) group or the phosphate group can be absent on one or both strands. This structure is the result of processing by dicer, an enzyme that converts either long dsRNAs or small hairpin RNAs into siRNAs. SiRNAs according to the present invention are linked to a hydrophobic polymer as defined above. A micelle according to the invention may comprise an amphiphilic block copolymer consisting of a hydrophobic polymer attached to the 3' or 5' end of a first nucleic acid molecule, wherein said first nucleic acid molecule is hybridized with a second nucleic acid molecule, and wherein the hybrid formed between said first and second nucleic acid is a siRNA. Accordingly, targeted delivery of siRNAs may be effected using the micelles of the invention.

Also other siRNA structures than those described above are also envisaged, provided they are capable of interfering with gene expression. Preferably, the double-stranded part has a length of about 12 to about 50 base pairs in length. The siRNA of the invention may either have overhanging sequences of up to 10 bases, preferably not more than 5 bases in length at either end or at one end, or may be blunt-ended. Also preferred is that the complementarity to the target gene extends over the entire length of the double-stranded part. The region which is complementary to the target gene is at least 12 bases, preferably at least 15, 16, 17, 18, 19, 20, 21, 22, 23 or more bases in length. The siRNA of the invention may be fully complementary to the target gene. Alternatively, the siRNA may comprise up to 5%, 10%, 20% or 30% mismatches to the target gene. Furthermore, siRNAs can be chemically modified e.g. on the backbone including the sugar residues. Preferred modifications of the siRNA molecules of he invention include linkers connecting the two strands of the siRNA molecule. Chemical modifications serve inter alia to improve the pharmacological properties of siRNAs and antisense RNAs such as in vivo stability and/or delivery to the target site within an organism. The skilled person is aware of such modified siRNAs as well as of means and methods of obtaining them, see, for example, Zhang et al., Curr Top Med Chem. 2006;6(9):893-900; Manoharan, Curr Opin Chem Biol. 2004 Dec;8(6):570-9.

Essentially any gene of which the sequence is known can thus be targeted based on sequence complementarity with an appropriately tailored siRNA. This has made siRNAs an important tool for gene function and drug target validation studies as well as for therapeutic intervention which is envisaged here.

The term "is hybridized with" is well known to the person skilled in the art and designates an interaction between nucleic acid strands by means of hydrogen bonds. The capability to hybridize requires at least partial complementarity between the interacting nucleic acid strands. The capability to hybridize may be assessed in hybridisation experiments. Hybridisation may involve the formation of a double strand between a first single-stranded nucleic and a second single-stranded nucleic acid or between a single-stranded part of a first nucleic acid and a single-stranded part of a second nucleic acid. In addition, hybridisation may also involve formation of hydrogen bonds between a first double-stranded nucleic acid and a second single-stranded nucleic acid or between a double-stranded part of a first nucleic acid a single-stranded part of a second nucleic acid. In the latter case, a triple helix may be formed. A triple helix may comprise non Watson Crick base pairs which in the art are also referred to as Hoogsteen base pairs.

It is well known in the art how to perform hybridization experiments with nucleic acid molecules. Correspondingly, the person skilled in the art knows what hybridization conditions s/he has to use to allow for a successful hybridization in,accordance with the present invention. The establishing of suitable hybridization conditions is referred to in standard text books such as Sambrook, Russell "Molecular Cloning, A Laboratory Manual"; Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). In one preferred embodiment, the hybridization is effected is under stringent conditions.

The term "stringent hybridization conditions" refers to conditions which comprise, e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 μg/ml denatured, sheared salmon sperm DNA, followed by washing the filters in 0.1 x SSC at about 65°C. Said conditions for hybridization are also known by a person skilled in the art as "highly stringent conditions for hybridization".

Also contemplated are lower stringency hybridization conditions ("low stringency conditions for hybridization"). Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37°C in a solution comprising 6X SSPE (20X SSPE = 3M NaCl; 0.2M $NaH_2PO4$; 0.02M EDTA, pH 7.4), 0.5% SDS, 30% formamide, 100 μg/ml salmon sperm blocking DNA; followed by washes at 50°C with 1X SSPE, 0.1% SDS. In addition, to achieve an even lower stringency, washes performed following stringent hybridization can be done at higher salt concentrations (e.g. 5X SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. The embodiment recited herein above preferably refers to highly stringent conditions and alternatively to conditions of lower stringency.

It is preferred that hybridisation occurs in the 3'-terminal segment of said first nucleic acid in case (a) of the main embodiment or in the 5'-terminal segment of said first nucleic acid in case (b) of the main embodiment. Full complementarity (i.e., no mismatches) over a length of at least 8, 9, 10, 11, 12, 13, 14 or 15 nucleotides of said first nucleic acid is preferred. Also longer complementary sequences are envisaged. The complementary nucleotides of said second nucleic acid are preferably located at the 3' end of said second nucleic acid in case (a) of the main embodiment and at the 5' terminus of said second nucleic acid in case (b) of the main embodiment.

Preferably, the micelles of the invention have a diameter in the range from 5 to about 20 nm. More preferred is a diameter between 10 and 15 nm.

The term "a targeting unit capable of selectively binding to a specific cell type and/or tissue" as used in accordance with the present invention refers to a moiety having the capacity to selectively associate with the specific target cell and/or tissue. Thus, the targeting unit facilitates specific delivery of the micelle of the invention to target cells and/or tissues while minimising any possible side effects resulting from delivery to non-target cells and/or tissues. In addition, the targeting unit can be used to achieve targeted delivery of the micelle of the invention to a cell and/or tissue whose location is unknown, diffuse and/or inaccessible. Targeting units include, but are not restricted to antibodies, ligands, substrates, nucleic acid molecules such as RNA, DNA, PNA or other molecules that bind specifically to a cell and/or tissue. The presence of two or more distinct types of targeting units on a micelle of the invention is deliberately envisaged. For example, two distinct antibodies or an antibody and folate (see below) may be present on one micelle of the invention. The use of a plurality of targeting units may further enhance specificity of delivery or may permit targeting of a broader range of cell types and/or tissues.

The targeting unit may be covalently attached to said second nucleic acid molecule. Attachment may be direct via a covalent bond formed between functional groups present on the targeting unit and the second nucleic acid molecule. Alternatively, attachment may involve a linker. The linker may be chosen to be a moiety capable of avoiding detection by the immune system. Such moieties are further detailed below. In a further alternative, the targeting unit may be non-covalently attached, for example by means of hybridisation in case the targeting unit is a nucleic acid, or by means of non-covalent attachment well known in the art such as the biotin-streptavidin system.

The invention includes micelles with one targeting unit. Preferably at least 2 and more preferred at least 3 targeting units are present in one micelle according to the invention. Deliberately envisaged are also higher numbers of targeting units in a single micelle according to the invention such as 5, 10, 15, 20, 25 or 30 targeting units. The skilled person, in view of the teaching of the present invention including the examples as well as his/her common general knowledge can determine without further ado the number of targeting units of a given type necessary to achieve a preset degree of specificity of delivery to the desired target cell types and/or tissues.

In the micelle of the invention, the targeting unit is located at the periphery. Thus, second nucleic acid molecules bound to a targeting unit via their 5' ends are hybridised with first nucleic acid molecules having the hydrophobic polymer attached to their 5' ends. The same considerations apply mutatis mutandis to case (b) of the main embodiment.

In order to produce cytotoxicity, most anticancer drugs require uptake into the cell. A number of mechanisms exist for the passage of drugs across the plasma membrane, including passive diffusion, facilitated diffusion, and active transport systems. Passive diffusion of drugs through the bilayer lipid structure of the plasma membrane is a function of the size, lipid solubility, and charge of the drug molecule. A further uptake mechanism is endocytosis. Endocytosis is a process whereby cells absorb material from the outside by engulfing it with their cell membrane. Endocytosis works with macromolecules or particulate matter beyond a certain size threshold. The mechanism of endocytosis is generally not available to most drugs which have a molecular with below 1000 Daltons. This is one of the reasons why formulation of drugs in the form of nanoparticles such as micelles has been considered. Without being bound by a specific theory, it is assumed that micelles of the invention are taken up by cells by an endocytotic mechanism.

Prior to actually entering a target cell, a nanoparticle or micelle has to be brought into the vicinity of the target cell. With many target cell types and tissues this is a challenging task. Delivery to target tissues is dependent on the permeability of blood vessels supplying the target tissue. For example, blood vessels supplying tumours have been observed to exhibit a certain degree of leakiness, i.e. the capability to permit escape of particles from the blood stream in a process known as the enhanced permeability and retention (EPR) effect (see, e.g. J. Controlled Release 1997, 43, 197-212).

Once the particles have accumulated in the target tissue via the above described EPR mechanism, the size of the particles affects how well the particles are delivered to the target cell, with smaller particles being more suitable for delivery than larger particles. Therefore, it is desirable to provide systems for targeted delivery which on the one hand benefit from the endocytotic mechanism of cellular uptake described above and on the other hand are small enough to successfully target individual cells once taken up into the tissue. The micelles according to the present invention are designed to fulfil these size requirements.

A further advantage of the present invention is that hybridisation between first and second nucleic acids provides the micelle according to the invention with a variety of possibilities regarding location and number of attached targeting units as well as diagnostic agents and hydrophilic drugs alone or in combination with each other (see also below). A variety of 5'- and 3'-modified nucleic acids bearing different functional groups are commercially available allowing several coupling strategies for a wide range of ligands, wherein for the purpose of the present invention the envisaged ligands include targeting units as well as diagnostic agents and hydrophilic drugs (for the latter see below). In contrast, functionalisation of conventional block copolymers with targeting moieties is demanding and typically requires multi-step synthesis and separation of ligand-modified from unmodified polymers. Typically, it has to be decided at the beginning of the synthesis of nanoparticles of the prior art which targeting moiety is to be used.

The amphiphilic block copolymers of the invention are furthermore synthesized in a fully automated fashion yielding structurally well-defined molecules because the nucleic acid segment is monodisperse and contains defined end groups. Such a highly-defined structure is an important criterion for approval of a drug or a delivery system. Likewise, the resulting spherical micelles exhibited a narrow size distribution.

Moreover, by employing negatively charged DNA with a persistence length of 50 nm as the hydrophilic block, surface exposition of the targeting moieties is guaranteed because, as is well accepted, the polymer chains of the corona in polyelectrolyte block copolymer aggregates are well-ordered and stretched. When a targeting unit is conjugated to other block copolymer systems, e.g. exhibiting a corona of polyethylene glycol, this is not achieved to the same extent. The term "persistence length" is well-known in the art and is a mechanical property quantifying the stiffness of a polymer. The persistence length is the length over which correlations in the direction of the tangent are lost, the direction of the tangent being determined for each segment or monomer of the polymer.

In a preferred embodiment the micelle of the invention comprises (a) an amphiphilic block copolymer consisting of a hydrophobic polymer attached to the 3' or 5' end of a nucleic acid molecule; (b) an amphiphilic block copolymer consisting of a hydrophobic polymer attached to the 3' or 5' end of a first nucleic acid molecule, wherein said first nucleic acid molecule is hybridized with a second nucleic acid molecule, wherein a hydrophilic drug is covalently attached via a cleavable linker to said second nucleic acid; (c) an amphiphilic block copolymer consisting of a hydrophobic polymer attached to the 3' or 5' end of a first nucleic acid molecule, wherein said first nucleic acid molecule is hybridized with a second nucleic acid molecule, wherein a diagnostic agent is covalently attached to said second nucleic acid; and/or (d) an amphiphilic block copolymer consisting of a hydrophobic polymer attached to the 3' or 5' end of a first nucleic acid molecule, wherein said first nucleic acid molecule is hybridized with a second nucleic acid molecule, wherein a moiety capable of avoiding detection by the immune system is covalently attached to said second nucleic acid.

Accordingly, micelles of the invention may further comprise amphiphilic block copolymers wherein the first nucleic acid molecule is not hybridised with a second nucleic acid molecule. Encompassed are micelles that contain 99% by weight of amphiphilic block copolymers consisting of a hydrophobic polymer attached to the 3' or 5' end of a nucleic acid molecule or 95%, 90%, 80%, 70%, 60%, 50%, 40%, 30%, 20%, 10%, 5% or 1% by weight of said amphiphilic block copolymers consisting of a hydrophobic polymer attached to the 3' or 5' end of a nucleic acid molecule.

A "cleavable linker" in accordance with the present invention is a linker which comprises or consists of a segment that is recognized and cleaved by enzymes, for example enzymes expressed in the extracellular space of the target tissue and/or in the cytoplasm of target cells. Examples for such linker encompass, but are not limited, to acid labile linkers. Acid labile linkers according to the invention include β-thiopropionate (M. Oishi et al Chembiochem 2005, 6, 718-725; M. Oishi et al Biomacromolecules 2003, 4, 1426-1432.); and phosphoramidate (J. H. Jeong et al Bioconjugate Chemistry 2003, 14, 473-479).

A "hydrophilic drug" in accordance with the present invention is a pharmaceutically active agent having a preference for polar environments. The term "hydrophilic" is defined herein above. As such, this embodiment of the present invention advantageously permits the delivery of hydrophilic drugs by means of a micelle carrying a targeting unit. Preferred hydrophilic drugs are hydrophilic cytotoxic agents. Cytotoxic agents are suitable for the treatment of conditions including cancer. Preferred/exemplary cytotoxic agents are provided further below.

The term "a diagnostic agent", as used in accordance with the present invention relates to an agent for diagnosing and/or monitoring a disease state. Preferred diagnostic agents are discussed below.

The term "moiety capable of avoiding detection by the immune system" refers to moieties known in the art and further detailed below which render a micelle less detectable or not detectable by the immune system, in particular by the cells of the reticuloendothelial system (RES). Such moieties are also referred to as "stealth moieties" or "moieties conferring stealth function".

Said hydrophilic drug, said diagnostic agent and said moiety capable of avoiding detection by the immune system are preferably attached to either the 3' or 5' end of said second nucleic acid.

In another preferred embodiment of the polymeric nanoparticle of the invention, the targeting unit is a ligand of a surface marker of said specific cell type and/or tissue type.

The term "ligand of a surface marker", as used in accordance with the present invention, is a molecule that is able to selectively bind to or form a complex with a surface marker. It binds to a site on the surface marker by intermolecular forces such as ionic bonds, hydrogen bonds and Van der Waals forces. The association is usually reversible and the ligand can dissociate, but may also be irreversible. Ligands include substrates, inhibitors, activators, and neurotransmitters. The interaction of ligands with their binding sites can be quantitatively characterized in terms of a binding affinity. Methods for testing affinity and selecting a suitable ligand are well-known to the person skilled in the art.

Regarding surface markers it is well-known in the art that gene expression varies at different stages in the development of cells (e.g. senescence, differentiation) and varies from cell type to cell type. Cell types have - due to differential expression - characteristic profiles of cell membrane constituents. The characteristic array of cell membrane constituents - also termed surface markers - can be used for classifying a cell, i.e. what kind of cell, what developmental stage, normal or diseased. Coating the surface of every cell in the body are specialized proteins, including receptors having the capability of selectively binding or adhering to other "signaling" molecules. There are many different types of receptors that differ in their structure and affinity for the signaling molecules. Normally, cells use these receptors and the molecules that bind to them as a way of communicating with other cells and to carry out their proper functions in the body. Each cell type, for example a liver cell, has a certain combination of receptors on their surface that makes them distinguishable from other kinds of cells. Examples of said surface markers include cluster of differentiation (CD) molecules on leukocytes and surface immunoglobulins. Antibodies specifically recognising CD molecules are deliberately envisaged as ligands of a surface marker according to the invention. It is further known that an increase, decrease, emersion or abolishment of certain surface markers correlates with certain diseases. For example, folate receptors (FRs) are highly expressed on the surface of various cancer cells and therefor emerged as new targets for specific localization of chemotherapeutics. The family of FRs currently consists of three known isoforms: FR$\alpha$, FR$\beta$ and FR$\gamma$ "L. Matherly, I.D. Goldman, in Vitamins and Hormones, Vol. 55 (Ed: G. Litwack), Academic Press, San Diego, CA 2003, 403". FR$\alpha$ is expressed primarily in cancer cells such as ovarian, mesothelioma, testicular, breast, colon, renal and malignant nasopharyngeal carcinomas. Folates, after binding to their receptors, are taken up by the cells via the receptor-mediated endocytic pathway. Any surface marker that alone or in combination with another surface marker is specific for a cell and/or tissue may be used as target for the targeting unit.

Accordingly, in a further preferred embodiment of the micelle of the invention, the ligand of the surface marker is selected from the group consisting of folate, transferrin, antiestrogens, estrogens, monoclonal antibody trastuzumab, neutravidin, saccharides, oligosaccharides and polysaccharides. Accordingly, the targeting unit of the invention may also act a pharmaceutically active agent. This applies, for example, to trastuzumab (Herceptin®).

The term "folate", as used in accordance with the present invention, relates to a water soluble B-vitamin. Folate is also referred to as vitamin $B_9$. In a preferred embodiment of the micelle according to the invention, wherein folate is said ligand of said surface marker, said micelle comprises at least 3, preferably 5, and more preferred 10 or more folate moieties.

The term "transferrin", as used in accordance with the present invention, relates to glycoprotein which binds iron as a blood plasma protein and thus is involved in cellular iron ion delivery; see e.g. Advanced Materials Volume 18, Issue 1, Pages: 80-83.

The term "antiestrogen", as used in accordance with the present invention, relates to all compounds that compete with estrogen for estrogen-receptor-binding sites. Therefore, the term "antiestrogen" includes fulvestrant, toremifene, tamoxifen, clomiphene or pharmaceutically acceptable salts thereof, such as, tamoxifen citrate or clomiphene citrate. Further antiestrogens are well-known to the person skilled in the art.

The term "estrogen", as used in accordance with the present invention, relates to compounds possessing estrogenic activity (cf. Fang et al. (2001), Chem Res Toxicol 14 (3):280-294). Encompassed are naturally, occurring estrogens, such as, for example, phytoestrogens, estradiol, estriol, estrone and synthetic compounds, i.e., xenoestrogens.

The term "saccharides" is well-known to those skilled in the art and includes oligosaccharides and polysaccharides. Saccharides are polycondensation products of sugars and include linear and branched saccharides.

In a more preferred embodiment of the micelle of the invention, the surface marker is selected from the group of folate receptors, transferrin receptors, epidermal growth factor receptors and cell-surface estrogen receptors.

Folate receptors specifically bind and internalize folate by endocytosis with subsequent recycling of the receptors to the membrane. As described above, folate receptors are over-expressed in a variety of cancer cells and therefore suitable as targets for locally confined chemotherapy.

Transferrin receptors are implicated in cellular iron import by binding transferrin loaded with iron and transporting it into the cell via internalization and subsequent vesicle formation. It is well-known that transferring receptors are over-expressed in a variety of cancers, for example, pancreatic cancer and neuroendocrine carcinaoma of the pancreas as well as breast cancer, cervical cancer and non-Hodgkin's cancer.

Epidermal growth factor receptors (EGFRs) belong to the ErbB family of receptors which is a subfamily of four related receptor tyrosine kinases: EGFR (ErbB-1), Her2/c-neu (ErbB-2), Her 3 (ErbB-3) and Her 4 (ErbB-4). Mutations affecting EGFR expression or activity have been proposed to promote the development of cancer as the EGFR signaling pathway is an important pathway regulating cellular growth; survival, proliferation and differention (Wiley et al., 2003; Trends Cell Biol 13:43-50). EGFRs are activated by ligand binding, which include epidermal growth factor (EGF) and transforming growth factor $\alpha$ (TNF$\alpha$) and neuregulins (NRGs).

Estrogenic steroids regulate cellular function in a variety of tissues and rapidly activate cell-surface estrogen receptors (ERs). Currently, two ERs have been identified, ER$\alpha$ and ER$\beta$. ER$\alpha$ is found for example in ovarian stroma cells, the hypothalamus and in breast cancer cells (ERs are overexpressed in around 70% of breast cancer cases).

The use of any of these surface markers may provide specific targeting of a variety of cells, in particular cancerous cells using the micelle of the invention when equipped with a cognate ligand as targeting unit. Further, any other suitable surface markers known or to be identified in the future as being specifically overexpressed or mutated in specific cells such as cancerous cells can be used as surface markers in accordance with the present invention.

In another preferred embodiment of the micelle of the invention, the targeting unit is an antibody, antibody fragment or aptamer.

The antibody, for example, can be polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

The antibody includes embodiments such as chimeric, single chain and humanized antibodies, as well as antibody fragments, like, inter alia, Fab fragments. Antibody fragments or derivatives further comprise $F(ab')_2$, Fv or scFv fragments; see, for example, Harlow and Lane (1988) and (1999), loc. cit. Various procedures are known in the art and may be used for the production of such antibodies and/or fragments. Thus, the (antibody) derivatives can be produced by peptidomimetics.

Further, techniques described for the production of single chain antibodies (see, inter alia, US Patent 4,946,778) can be adapted to produce single chain antibodies specific for polypeptide(s) and fusion proteins of this invention. Also, transgenic animals may be used to express humanized antibodies specific for surface markers. Most preferably, the antibody is a monoclonal antibody. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a protein (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, amongst others, viruses or plasmid vectors.

The antibody described in the context of the invention is capable to specifically bind/interact with an epitope of surface markers or other proteins. The term "specifically binding/interacting with" as used in accordance with the present invention means that the antibody does not or essentially does not cross-react with an epitope of similar structure. Cross-reactivity of a panel of antibodies under investigation may be tested, for example, by assessing binding of said panel of antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or do not essentially bind to any of the other epitope are considered specific for the epitope of interest and thus to be antibodies in accordance with this invention. Corresponding methods are described e.g. in Harlow and Lane, 1988 and 1999, loc cit.

The antibody specifically binds to/interacts with conformational or continuous epitopes which are unique for surface receptors or other proteins. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues which are present in a single linear segment of a polypeptide chain.

Aptamers can be DNA or RNA aptamers or peptide aptamers. DNA or RNA aptamers consist of strands of oligonucleotides and can be of varying length. Peptide aptamers may consist of a variable peptide loop attached at both ends to a protein scaffold. The aptamers, as used in accordance with the present invention, specifically bind to surface markers or other proteins and/or interfere with the function of said surface markers or other proteins. Methods for production of specifically binding aptamers are well-known to the person skilled in the art and described, for example, in Ellington et al., Nature 1990, 346(6287:818-822; Bock et al., Nature 1992, 355(6360):564-566; or Cohen et al., PNAS 1998, 95(24):14272-14277.

In another preferred embodiment, said said moiety capable of avoiding detection by the immune system is selected from polyethylene glycol, poloxamines and poloxamers. These moieties exert stealth function as further explained herein.

A major problem with in vivo delivery of any residue attached to a polymeric nanoparticle is that after administration said polymeric nanoparticle may be captured by the defence system of the body. It is known that the capture of nanoparticles can be minimised and the half-life increased by the attachment of hydrophilic moieties to the surface of particles (cf. U.S. Pat. No. 4,904,479). This strategy is known as the "stealth liposome concept" and consequently any particles including micelles designed accordingly have stealth function. Any ligand conferring stealth function to the polymeric nanoparticle of the invention can be attached as long as it does not significantly change other preferred properties of said polymeric nanoparticle. Preferred moieties are polyethylene glycol (PEG, cf. U.S. Pat. No. 4,904,479), poloxamines or poloxamers. Poloxamers ("Pluronic") or poloxamines ("Tetronic") are manufactured and commercially available from BASF Wyandotte Corporation, for example.

In another preferred embodiment said specific cell type and/or tissue is associated with a disease.

A cell and/or tissue associated with a disease may be any cell and/or tissue displaying characteristics of a disease. Characteristics of diseased cells and/or tissues are well-known to the skilled person and several methods to determine said characteristics are known in the art, such as, for example, visual assessment by any form of microscopy (e.g. in combination with molecular biology techniques like FISH) or assessment of the genetic makeup by gene micro array, gene expression studies such as real-time PCR and Western blotting.

According to the present invention it is envisaged that the micelle of the invention may be, for example, used as vehicle for target specific drug delivery. The targeting unit of the micelle may be directed at the diseased cell and/or tissue and the binding and subsequent internalization may restore normal function of the diseased cell and/or tissue relative to a control cell and/or tissue. This may lead to improvement of, for example, the clinical outcome of a patient.

In a more preferred embodiment, said specific cell type and/or tissue is a tumor cell.

It is preferred that the micelle targets a cell displaying characteristics of a cancerous cell. Methods to identify cells associated with a disease such as cancer have been described supra and are well-known to the person skilled in the art. Targeting of the cell by the micelle may be accomplished by using, for example, the ligands and surface markers described hereinabove.

In a further preferred embodiment said specific cell type and/or tissue is a human cell type and/or tissue.

In another preferred embodiment of the invention, said hydrophilic drug is selected from the group consisting of topotecan, irinotecan, bleomycin, doxorubicin hydrochloride and mitomycin.

The hydrophilic drug attached to said second nucleic acid molecule is preferably topotecan or irinotecan, which are both topoisomerase I inhibitors, or bleomycin, mitomycin and doxorubicin hydrochloride, all of which are anticancer/cytotoxic antibiotics. These drugs have shown to be particularly useful as anticancer drugs and further, because of their hydrophilic nature are preferred hydrophilic drugs to be used in accordance with the present invention.

In a preferred embodiment said diagnostic agent is selected from the group consisting of folate-based radiodiagnostics, gallium-based radiodiagnostics, indium-based radiodiagnostics, technetium-based radiodiagnostics and near-infrared excitable fluorescent agents.

Radiodiagnostics are radioactively labeled compounds which are used as tracers in the diagnosis of a variety of diseases in the field of nuclear medicine. The same compounds may also be used for treatment of said diseases. In this case said compounds are also referred to as radiopharmaceuticals. Suitable radioisotopes which can be used to manufacture radiodiagnostics and radiopharmaceuticals are well-known in the art and include, for example, Calcium-47, Carbon-14, Fluorine-18, Gallium-67, Indium-111, Iron-59, Technetium-99m and Thallium-201. Any radiodiagnostics may be used in accordance with the present invention. Preferred are folate-based radiodiagnostics, gallium-based radiodiagnostics, indium-based radiodiagnostics or technetium-based radiodiagnostics. The skilled person is aware of near-infrared excitable fluorescent agents. Examples include indocyanine green, indodicarbocyanine, indotricarbocyanine and IRDye 780.

In another preferred embodiment of the invention, the micelle of the invention further comprises a hydrophobic drug.

Hydrophobic drugs as used in accordance with the present invention are any hydrophobic drugs that can be loaded into the hydrophobic core of the micelle of the invention. Methods of loading micelles and determination of loading content are known to the skilled person and are described, for example, in Shuai et al., J. Controlled Release (2004), 98, 415. Preferred hydrophobic drugs are hydrophobic cytotoxic agents. Preferred/exemplary cytotoxic agents are provided further below.

Advantages of a micelle of the present invention loaded with a hydrophobic drug include the following. Anticancer drugs, such as doxorubicin, if administered systematically have severe side-effects and thus possibilities of treatment of cancer with said drugs is often limited as regards dose and period of treatment in view of medical considerations of side-effects and predisposition to organ failure etc. The use of micelles of the invention minimizes the systemic circulation of said drugs. Exposure to drugs is limited to cancer cells due to target specificity and intracellular delivery of the drugs by means of the micelles. It follows that with less drug the same or better results can be achieved.

Furthermore, the transport of drugs - in particular anti-cancer drugs - into target cells is frequently hampered due to multi-drug resistance efflux pumps (e.g. P-glycoprotein) that actively secrete xenobiotics from the cell interior to the extracellular space. This results in low cellular drug concentrations such that the chemotherapeutic agents given in therapeutic doses will not kill the tumor cells (O'Connor R., 2007, Anticancer Res. 27: 1267-72; P. Anderle et al., 1998, J. Pharm. Sci.: 87: 757-762; S. Döppenschmitt et al., 1998, Pharm. Res. 15: 1001-1006). Thus, entrapment of a drug in the hydrophobic core of the micelle of the invention as described herein will enhance the cellular permeability and uptake of the drug into the target cell and bypass the permeability-limiting efflux mechanism. Thus the micellar delivery system described here will allow to circumvent the phenomenon of "multi-drug resistance" which limits the clinical utility of many anti-cancer drugs.

Accordingly, in a more preferred embodiment of the invention, the hydrophobic drug is selected from the group consisting of Altretamine, Bexarotene, Methotrexate, Trimetrexate, Edatrexate, Piritrexim, Paclitaxel, Docetaxel, Tripentones, Doxorubicin, Bicalutamide and Cisplatin. Methotrexate, Trimetrexate, Edatrexate and Piritrexim are antifolates. Antifolates are substances which block the activity of folic acid. Antifolates are established cancer therapeutics. Bicalutamide is an oral non-steroidal anti-endogen for the treatment of cancers such as prostate cancer. Cisplatin is one of several platinum containing agents used in the treatment of cancer. Further examples of this class of compounds include Carboplatin and Oxaliplatin.

In another preferred embodiment of the polymeric nanoparticle of the invention, the nucleic acid molecules are oligonucleotides or siRNAs.

Oligonucleotides include oligodeoxyribonucleotides and oligoribonucleotides. Oligodeoxyribonucleotides (oligodeoxynucleotides, ODNs) are polymers exclusively or predominantly consisting of monomers of deoxynucleotides. Analogously, oligoribonucleotides are polymers exclusively or predominantly consisting of monomers of ribonucleotides. Oligonucleotides can be of different length depending on the number of linked monomers. Methods to design and manufacture oligonucleotides are well-known to the person skilled in the art. Preferably, the length of the oligodeoxynucleotides according to the invention is between 8 and 40, more preferred between 10 and 30 and yet more preferred between 15 and 25 nucleotides. Any integer number falling into these ranges is also deliberately envisaged. Accordingly, oligonucleotides according to the invention may inter alia have a length of 16, 17, 18, 19, 20, 21, 22, 23 or 24 nucleotides.

Preferred oligonucleotides according to the invention are immune stimulating oligonucleotides. In a more preferred embodiment a micelle of the invention comprises as first and/or second nucleic acid one or more copies of one or more of the oligoribonucleotides comprising or consisting of a sequence set forth in any one of SEQ ID NOs. 1 to 21 and oligodeoxynucleotides comprising or consisting of a sequence set forth in any one of SEQ ID NOs. 22 to 27. The sequences of SEQ ID NOs. 1 to 18 and 22 to 27 have been reported in Journal of Experimental Medicine, Vol. 202, 1575-1585 (2005). The sequences of SEQ ID NOs. 19 to 21 have been reported in Science, Vol. 303, 1526-1529 (2004). Such micelles exert an immune stimulating effect upon delivery to the target cell type or target tissue.

SiRNAs are further described herein above.

In a preferred embodiment, the hydrophobic polymer of the polymeric nanoparticle of the invention is selected from the group consisting of polypropylene oxide, poly(D,L-lactic-co-glycolic acid), polybutadiene and polyisoprene.

Polypropylene oxide (PPO) is a polymer with proven biocompatibility toward different cell types when administered as a constituent component of amphiphilic block copolymer micelles (see, e.g., Miller, D.W. et al., 1997, Bioconjugate Chem. 8:649). Hydrophobic polymers according to the invention may be biodegradable polymers such as polylactic-co-glycolic acid or polymers which are not biodegradable or only to a limited extent such as polypropylene oxide.

The invention also provides a composition comprising the micelle of the invention.

The term "composition", as used in accordance with the present invention, relates to a composition which comprises at least one of the polymeric nanoparticles of the invention. It may, optionally, comprise further molecules capable of altering the characteristics of the polymeric nanoparticles of the invention thereby, for example, reducing, stabilizing, delaying, modulating and/or activating their function. The composition may be in solid, liquid or gaseous form and may be, inter alia, in the form of (a) powder(s), (a) tablet(s), (a) solution(s) or (an) aerosol(s). In a more preferred embodiment, the composition is a pharmaceutical composition optionally further comprising a pharmaceutically acceptable carrier, excipient and/or diluent.

In accordance with the present invention, the term "pharmaceutical composition" relates to a composition for administration to a patient, preferably a human patient. The pharmaceutical composition of the invention comprises the compounds, i.e. micelles, recited above. The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutical carriers are well known in the art and include sodiumchloride solutions, phosphate buffered sodiumchloride solutions, water, emulsions, such as oil/water emulsions, various types of wetting agents, sterile solutions, organic solvents including DMSO etc. Preferably the carrier is a parenteral carrier, more preferably a solution that is isotonic with the blood of the recipient. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) (poly)peptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The term "parenteral" as used herein refers to modes of administration which include intravenous, intramuscular, intraperitoneal, intrasternal, subcutaneous and intraarticular injection and infusion.

Compositions comprising such carriers can be formulated by well known conventional methods. Generally, the formulations are prepared by contacting the components of the pharmaceutical composition uniformly and intimately with liquid carriers or finely divided solid carriers or both. Then, if necessary, the product is shaped into the desired formulation.

These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgment of the ordinary clinician or physician. Generally, the regimen as a regular administration of the pharmaceutical composition should be in the range of 1 $\mu$g to 20 g units per day. However, a more preferred dosage might be in the range of 0.01 mg to 100 mg, even more preferably 0.01 mg to 50 mg and most preferably 0.01 mg to 10 mg per day. Administration of pharmaceutical compositions of the invention may be effected by different ways, e.g., by intravenous, intraperitoneal, subcutaneous, intramuscular, topical, intradermal, intranasal or intrabronchial administration.

The components of the pharmaceutical composition to be used for therapeutic administration must be sterile. Sterility is readily accomplished by filtration through sterile filtration membranes (e.g., 0.2 micron membranes).

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection. Preservatives and other additives may also be present such as, for example, antimicrobials, anti oxidants, chelating agents, and inert gases and the like. Furthermore, the pharmaceutical composition may comprise further agents depending on the intended use of the pharmaceutical composition.

The pharmaceutical composition may be particularly useful for the treatment of diseases, preferably, diseases selected from neurodegenerative diseases, hepato-biliary diseases, cardiovascular diseases or pulmonary diseases.

In another preferred embodiment the composition of the invention is a diagnostic composition.

In accordance with the present invention, the term "diagnostic composition" relates to compositions for diagnosing individual patients for their potential response to or curability by the pharmaceutical compositions of the invention. The diagnostic composition of the invention comprises the diagnostic agents recited above. The diagnostic composition may further comprise an appropriate carrier, diluent or excipient. The diagnostic compositions may be packaged in a container or a plurality of containers.

The invention also provides a method of killing a specific target cell and/or tissue, the method comprising exposing the specific target cell and/or tissue to the micelle of the invention or to the pharmaceutical composition of the invention wherein said targeting unit and/or said hydrophilic drug, if present, and/or said hydrophobic drug, if present, is/are (a) cytotoxic agent. Said method may be performed *in vitro, ex vivo* or *in vivo.*

The micelle or the pharmaceutical composition containing said micelle may be used to directly or indirectly induce cell death of a specific target cell and/or tissue. Directly inducing cell death is achieved by a micelle or composition of the invention comprising a cytotoxic compound which is released upon internalization or when the micelle is in close proximity to the target cell. The cytotoxic mechanism of cytotoxic agents may be, for example, DNA intercalation, crosslinking DNA, cell cycle arrest, lytic activity, and/or inhibition of metabolism (e.g., folic acid metabolism). Cytotoxicity can be measured, for example, by the MTT assay, Trypan blue assay, Sulforhodamine B assay, WST assay or clonogenic assay. Indirectly inducing cell-death can be achieved, for example, by tagging cells thus making them prone for drug-mediated or immune system-mediated cell-death. A suitable method is, for example the antibody-dependent cell-mediated cytotoxicty (ADCC) approach, wherein the target cell is tagged with an antibody. Being thus marked, the cell can be recognized by lymphocytes and killed.

The following table provides classes, subclasses and exemplary/preferred representatives of cytotoxic compounds which are envisaged for practicing the present invention. Hydrophobic cytotoxic agents may be used as hydrophobic drugs as recited herein above. Hydrophilic cytotoxic agents may be linked via cleavable linker to a second nucleic acid as defined herein above.

**Table 1.** Examples of suitable cytotoxic agents.

| | | |
|---|---|---|
| Alkylating agents | Nitrogen mustards | Chlorambucil, Chlormethine, Cyclophosphamide, Ifosfamide, Melphalan |
| | Nitrosoureas | Carmustine, Fotemustine, Lomustine, Streptozocin |
| | Platinum containing agents | Carboplatin, Cisplatin, Oxaliplatin, BBR3464 |
| | | Busulfan, Dacarbazine, Mechlorethamine, Procarbazine, Temozolomide, Thiotepa, Uramustine |
| Antimetabolites | Folic acid | Aminopterin, Methotrexate, Pemetrexed, Piritrexim, Raltitrexed, Trimetrexate, Edatrexate |
| | Purine | Cladribine, Clofarabine, Fludarabine, Mercaptopurine, Pentostatin, Thioguanine, |
| | Pyrimidine | Capecitabine, Cytarabine, Fluorouracil, Floxuridine, Gemcitabine |
| Spindle poison plant alkaloids | Taxane | Docetaxel, Paclitaxel |
| | Vinca | Vinblastine, Vincristine, Vindesine, Vinorelbine |
| Cytotoxic/antitumor antibiotics | Anthracycline | Daunorubicin, Doxorubicin, Epirubicin, Idarubicin, Mitoxantrone, Valrubicin |
| | | Bleomycin, Hydroxyurea, Mitomycin, Actinomycin |
| Topoisomerase inhibitors | Camptotheca | Camptothecin, Topotecan, Irinotecan |
| | Podophyllum | Etoposide, Teniposide |
| Monoclonal antibodies | | Alemtuzumab, Bevacizumab, Cetuximab, Gemtuzumab, Panitumumab, Rituximab, Tositumomab, Trastuzumab |
| Photosensitizers | | Aminolevulinic acid, Methyl aminolevulinate, Porfimer sodium, Verteporfin |
| Kinase inhibitors | | Dasatinib, Erlotinib, Gefitinib, Imatinib, Lapatinib, Nilotinib, Sorafenib, Sunitinib, Vandetanib |

(continued)

| | | |
|---|---|---|
| Humanized monoclonal antibodies | | Alemtuzumab, Apolizumab, Aselizumab, Atlizumab, Bapineuzumab, Bevacizumab, Bivatuzumab mertansine, Cantuzumab mertansine, Cedelizumab, Certolizumab pegol, Cidfusituzumab, Cidtuzumab, Daclizumab, Eculizumab, Efalizumab, Epratuzumab, Erlizumab, Felvizumab, Fontolizumab, Gemtuzumab ozogamicin, Inotuzumab ozogamicin, Labetuzumab, Lintuzumab, Matuzumab, Mepolizumab, Motavizumab, Natalizumab, Nimotuzumab, Nolovizumab, Numavizumab, Ocrelizumab, Omalizumab, Palivizumab, Pascolizumab, Pecfusituzumab, Pectuzumab, Pertuzumab, Pexelizumab, Ralivizumab, Ranibizumab, Reslivizumab, Reslizumab, Resyvizumab, Rovelizumab, Ruplizumab, Sibrotuzumab, Siplizumab, Sontuzumab, Tacatuzumab tetraxetan, Tadocizumab, Talizumab, Tefibazumab, Tocilizumab, Toralizumab, Trastuzumab, Tucotuzumab celmoleukin, Tucusituzumab, Umavizumab, Urtoxazumab, Visilizumab |
| Other | | Alitretinoin, Altretamine, Amsacrine, Anagrelide, , Asparaginase, Bexarotene, Bortezomib, Denileukin diftitox, Estramustine, Hydroxycarbamide, Masoprocol, Mitotane, Pegaspargase, Tretinoin |

Further, the present invention relates to the use of the micelle of the invention for the preparation of a pharmaceutical composition for the treatment of cancer, neurodegenerative diseases, hepato-biliary diseases, cardiovascular diseases or pulmonary diseases. Both hydrophobic drugs and hydrophilic drugs may be constituents of the micelles of the invention (see above). Exemplary suitable drugs for treatment of the respective diseases are provided below.

<u>Neurodegenerative diseases:</u>

Acetylcholinesterase inhibitors:

- Tetrahydroaminacridine
- Donepezil hydrochloride
- Rivastigmine
- Galantamine
- Metrifonate

Chelators:

- D-penicillamine
- Trientine
- Bathocuproine
- Desferoxamine

<u>Gastrointestinal and hepato-biliary diseases:</u>

- Anticancer agents in liver malignancy

- In viral hepatitis: Antiviral agents such as lamivudine, ribavirin, IFNα

For the purpose of gene delivery in gene therapy, micelles can be engineered to display the hepatitis B virus surface L antigen (HBsAg) on their surface. Such micelles are devoid of viral genomes.

Cardivascular diseases:

- Dexamethasone
- Digoxin
- ACE (Angiotensin converting enzyme) inhibitors
- Beta blockers
- Sodium channel blockers
- Calcuim channel blockers

Pulmonary diseases:

- $\beta_2$-agonists such as salbutamol, albuterol, terbutaline, formoterol
- Corticosteroids such as budesonide, flixotide, beclomethasone

The term "cancer", in accordance with the present invention refers to a class of diseases or disorders characterized by uncontrolled division of cells and the ability of these to spread, either by direct growth into adjacent tissue through invasion, or by implantation into distant sites by metastasis (where cancer cells are transported through the bloodstream or lymphatic system).

The term "neurodegenerative disease", in accordance with the present invention refers to a class of diseases or disorders wherein neurons deteriorate and due to the inability of the body to regenerate neurons (except a small number neural stem cells) the cells for example of the brain or spinal chord cannot be adequately regenerated. Symptoms encompass ataxia as well as dementia in affected individuals.

The term "Gastrointestinal and hepato-biliary disease", in accordance with the present invention relates to diseases or disorders affecting the liver, gall bladder and bile ducts. Such diseases and disorders include, for example, cirrhosis, hepatitis, virally induced hepatitis, liver tumors, fatty liver, polycystic liver, Morbus Crohn, Colitis ulcerosa and cholangiocarcinoma.

The term "cardiovascular disease", in accordance with the present invention relates to a class of diseases or disorders involving the heart an/or blood vessels.

The term "pulmonary diseases", as used in accordance with the present invention relates to diseases affecting the respiratory system and can be classified into obstructive, i.e. impeding the rate of low into and out of the.lungs, and restrictive, i.e. reduction in the functional volume of the lungs, conditions. Such diseases include, for example, asthma, bronchitis, asbestosis, fibrosis, sarcoidosis, lung cancer, pneumonia, pulmonary edema and pulmonary hypertension.

Furthermore, the invention relates to a kit comprising the micelle of the invention.

The kit of the invention may contain further ingredients such as other pharmaceutical or diagnostic agents or selective media. The kit of the invention can be used for carrying out a method of the invention and can be, inter alia, employed in a variety of applications, e.g., in the diagnostic field, as research tool or in the therapeutic field. The parts of the kit of the invention can be packaged individually in vials or other appropriate means depending on the respective ingredient or in combination in suitable containers or multicontainer units. Manufacture of the kit follows preferably standard procedures which are known to the person skilled in the art. The kit may be used for methods for detecting diseases or for treating a disease in accordance with any one of the above-described methods of the invention, employing, for example, immunoassay techniques such as radioimmunoassay or enzyme immunoassay techniques such as those described herein before and in the

Examples.

The Figures show:

**Figure 1.** Characterization of DNA block copolymers by (A) MALDI-TOF mass spectrometry and (B) PAGE.

**Figure 2.** The MALDI-TOF mass spectrum of ssDNA-FA conjugate (Found: 7385 g/mol calculated: 7391 g/mol)

**Figure 3.** Analysis of ssDNA and its folic acid conjugate in a 20 % polyacrylamide gel.

**Figure 4.** Correlation function of the PPO-b-DNA diblock copolymers with increasing FA moieties (A) at the core and (B) at the corona of the micelle.

**Figure 5.** Ethidium bromide-stained agarose gel of PCR products. P27: Lane 1, RFC; lane 2, FRα; lane 3, FRβ and P62: lane 4, RFC; lane 5, FRα; lane 6, FRβ. The gel electrophoresis of the PCR product clearly showed the similarity of younger and older passage by means of the three folate transport routes.

**Figure 6.** Relative gene expression levels $(2^{-\Delta\Delta C_T})$ of FRα and FRβ to RFC which were normalized to GAPDH in Caco-2 cells. Values are shown as mean of three different reactions ± SD.

**Figure 7.** Viabilities of Caco-2 cell monolayers exposed to different folic acid conjugated nanoparticles. Out 28: DNA block copolymer micelles with 28 targeting units at the periphery of the micelle, Out 11: DNA block copolymer micelles with 11 targeting units at the periphery of the micelle, Out 2: DNA block copolymer micelles with 2 targeting units at the periphery of the micelle, In 28: DNA block copolymer micelles with 28 targeting units in the core of the micelle, In 11: DNA block copolymer micelles with 11 targeting units in the core of the micelle and In 2: DNA block copolymer micelles with 2 targeting units in the core of the micelle. Values are means of triplicates ± SD.

**Figure 8.** Uptake of folic acid linked micelles into human Caco-2 monolayers incubated for 3 h (Fig. 8A). Out 28: DNA block copolymer micelles with 28 targeting units at the periphery of the micelle, Out 11: DNA block copolymer micelles with 11 targeting units at the periphery of the micelle, Out 2: DNA block copolymer micelles with 2 targeting units at the periphery of the micelle, In 28: DNA block copolymer micelles with 28 targeting units in the core of the micelle, In 11: DNA block copolymer micelles with 11 targeting units in the core of the micelle and In 2: DNA block copolymer micelles with 2 targeting units in the core of the micelle. Results are shown as the average values of triplicates ± SD. CLSM image of the uptake of labeled micelles inside Caco-2 cells (Fig. 8B).

**Figure 9.** Schematic representation of drug delivery system based on DNA block copolymers loaded with a hydrophobic drug (Fig. 9A). The viability of cells after incubation with A) Dox-loaded micelles covalently linked to targeting unit B) Dox-loaded micelles with but not covalently linked to folic acid C) Dox-loaded micelles D) folic acid conjugated micelles in the absence of dox (Fig. 9B).

The invention will now be described by reference to the following examples which are merely illustrative and are not to be construed as a limitation of scope of the present invention.

**Materials and Methods**

Unless otherwise stated, materials were obtained from commercial suppliers and used without further purification. The poly(propyleneglycol) monobutyl ether, N-diisopropyl-2-cyanoethyl-chlorophosphoramidite, diisopropylethylamine were purchased from Aldrich. The dimethoxytrityl (DMTr) protected phosphoramadites were purchased from Proligo (Germany). DNA block copolymers were synthesized using AKTA Oligopilot (Amersham Biosciences, Sweden). Tetramethylenesilane and triphenylphosphine were used as the references for the $^{1}H$ NMR and $^{31}P$ NMR spectra, respectively. The spectra were recorded on Bruker AMX 250 (250MHz) or DRX 500 (500 MHz) spectrometers. Molecular weights were determined using matrix-assisted laser desorption/ionisation time-of-flight (MALDI-TOF), and the spectra were recorded on a Bruker MALDI-TOF (Reflex-TOF) mass spectrometer. Size exclusion chromatography (SEC) analysis was done in THF against polystyrene standard using a Soma UV/ERC RI detector and a pump from Waters Corporation (USA). Confocal laser scanning microscopy measurements were carried out with a LSM 510 laser scanning module coupled to a Zeiss Axiovert 200M inverted microscope. In all experiments, MilliQ standard water (Millipore Inc., USA) with a typical resistivity of 18.2MΩ/cm was used. Spectrophotometric assays and fluorescence measurements were conducted using a SpectraMax M2 plate reader (Molecular Devices, USA). Oligonucleotides were quantified spectrophotometrically at a wavelength of 260 nm and by denaturing polyacrylamide gel electrophoresis (PAGE) followed by staining with ethidium bromide and UV transillumination. The densiometric quantification was determined using the GelPro programme distributed from Intas GmbH (Germany). Caco-2 cells were obtained from DSMZ (Deutsche Sammlung von Mikroorganismen und Zellkulturen GmbH, Braunschweig, Germany). All the cell culture media and supplements were purchased from Biochrom AG (Berlin, Germany). HEPES was provided from Merck (Darmstadt, Germany). RNA STAT-60™ was purchased from Tel-Test Inc. (Friendswood, TX, USA). DNA-free™ purification kit was obtained from Ambion Ltd. (Cambridgeshire, UK). SuperScript™ First-Strand Synthesis System for RT-PCR, sense, and antisense primers were purchased from Invitrogen™ Ltd. (Paisley, UK). Expand™ High Fidelity PCR System Kit was provided from Roche Diagnostics GmbH (Mannheim, Germany). GeneRuler™ was purchased from Fermentas GmbH (Leon-Rot, Germany). QuantiTect Probe RT-PCR Kit and RNeasy Mini Kit were from Qiagen GmbH (Hilden, Germany). Sense and antisense primers and TaqMan probes for real-time PCR were purchased from Operon Biotechnologies (Cologne, Germany).

**Example 1: Synthesis of polymeric nanoparticles**

DNA-*b*-PPO Diblock Copolymers

The preparation of ssDNA-b-PPO copolymers, and the formation of micelles were carried out as described previously (Alemdaroglu, F. E. et al., 2006, Angew. Chem., Int. Ed. 45:4206). Oligonucleotides were quantified spectrophotometrically at a wavelength of 260 nm. Briefly, a phosphoramidite-functionalized PPO (Mn = 6800 g/mol) was synthesized and attached to the 5' terminus of the nucleic acid fragment (5'-CCTCGCTCTGCTAATCCTGTTA-3', 22mer, Mw = 6700 g/mol, SEQ ID NO: 28) via automated solid phase synthesis. The resulting block copolymer was analyzed and purified by denaturing polyacrylamide gel electrophoresis (PAGE; see Figure 1A) and the molecular weight confirmed by MALDI-TOF mass spectrometry (Figure 1 B). Dynamic Light Scattering (DLS) measurements of the DNA block copolymer aggregates revealed the formation of uniform micelles of diameter 10.8 $\pm$ 2.2 nm.

Synthesis of Oligonucleotide-Folic Acid Conjugates

For equipping these micelles with targeting units, 5'- and 3' -modified ODNs that encode the complementary sequence of DNA-*b*-PPO were reacted with folic acid (FA). In detail, the synthesis of ssDNA-folic acid (FA) conjugates was carried out by mixing e.g. 5'-amino-modified oligonucleotide (TAACAGGATTAGCAGAGCGAGG, 22mer, Mw = 6950 g/mol, SEQ ID NO: 29) (30 $\mu$mol) with folic acid (100 $\mu$mol) in the presence of 4-(4,6-dimethoxy-1,3,5-triazin-2-yl)-4-methylmorpholinium chloride (DMT-MM) (35$\mu$mol) in 1 ml of water. The mixture was allowed to react for 12 h at room temperature. The conjugate was purified using 20 % denaturing PAGE. After excision the bands were dialyzed against water for 24 hours. Subsequently, the conjugates were lyophilized yielding 60% ssDNA-FA conjugate. Characterization of the products was carried out by MALDI-TOF mass spectrometry (Figure 2) and PAGE (Figure 3). These conjugates can be hybridized with the micelles so that the FA is either positioned at the periphery (5') or in the core (3') of the nanoparticle.

Functionalization of Micelles with Folic Acid

In order to study the effect of FA density and position within the nanoparticles on the targeting efficiency, DNA-b-PPO copolymers were hybridized in different ratios with the targeting unit-bearing oligonucleotides. This convenient procedure resulted in micelles with on average 2, 11 or 28 (fully hybridized) FAs either at the periphery or at the hydrophobic-hydrophilic interface of the micelles. The hybridization was carried out by dissolving ssDNA-b-PPO diblock copolymer and the ssDNA-FA conjugate in TAE buffer (20 mM tris(hydroxymethyl)aminomethane-HCl, pH 8.0; 10 mM acetic acid, 0,5 mM EDTA) containing $Na^+$ (100 mM) and $Mg^{2+}$ (60 mM). The mixture was heated to 95°C and was slowly cooled to room temperature over the course of 3 days (1 degree per hour) by using a polymerase chain reaction (PCR) thermocycler (Biorad, USA). The final concentration of DNA-b-PPO was between 200-500 $\mu$M.

Characterization of DNA-PPO block copolymer Micelles by Dynamic Light Scattering

The effective hydrodynamic diameter of the micelles was measured by dynamic light scattering (DLS) at 25 °C using a dynamic light scattering photometer (ALV 5800, Avalanche Photodiode) equipped with He-Ne laser at a wavelength of 632 nm. Maintenance of the narrow size distribution of the micelles was observed. Moreover, the diameter of the micelles was found to increase slightly with an increase in the number of FA units. For 2, 11 and 28 FA moieties at the rim, micelle diameters of 11.2 $\pm$ 1.6 nm, 13.2 $\pm$ 2.4 nm and 14.4 $\pm$ 2.2 nm were measured, respectively. When FA is positioned inside, diameters of 11.2 $\pm$ 1.8 nm, 12.2 $\pm$ 2.4 nm and 12.2 $\pm$ 2.0 nm were detected for the same FA densities. Importantly, the nanoparticles were on the order of 10 nm, an important design criterion for efficient tumor cell-specific delivery.

Data were gathered and processed using the ALV 5000/E software. The samples were prepared in buffer medium and measured at a concentration of 2 mg/ml. For each micelle system the measurements were carried out in triplicate.

**Example 2: Quantification of Relative Gene Expression Levels of Folate Receptors in Caco-2 cells**

Human colon adenocarcinoma (Caco-2) cells were employed as a cancerous cell line to study the uptake of the differently decorated DNA block copolymer micelles. Firstly, the availability of three known genes for folic acid transport, i.e. RFC, FRα and FRβ, was examined in these cells and their relative gene expression levels were measured by real-time polymerase chain reaction (PCR).

Preparation of the Caco-2 Monolayers

Caco-2 cells (passage 27, 54 and 62) were split and seeded into 24-well plates with a density of 100,000 cells/well. The medium was changed three times a week. The development of the monolayers was examined under the microscope until the $16^{th}$ day. Total cellular RNA both for RT-PCR and for real-time PCR was isolated from Caco-2 monolayers on the $16^{th}$ day post-seeding.

Isolation of Total Cellular RNA, Reverse Transcriptase Reaction, PCR and Gel Electrophoresis

For the investigation of some of the known transport routes of folates into the cells, and additionally the effect of passage number on the expression of the transport systems, one younger (passage 27) and one older (passage 62) passage was used. The RNA was isolated from the cells using RNA STAT-60™ according to the company's protocol for RNA isolation. The obtained RNA pellet was dried by airdrying. 25 μl of RNase-free water was added to dissolve the RNA and was purified using a DNA-free™ purification kit.

The integrity of the isolated RNA was checked by standard gel electrophoresis with 1% agarose. The total RNA was reversely transcribed into cDNA by using SuperScript™ First-Strand Synthesis System for RT-PCR according to the manufacturer's guidance. cDNA obtained after reverse transcription was then amplified by PCR. The sequences of primers used in this study are shown in Table 2.

**Table 2.** The sequences of the sense and antisense primers for RFC, FRα and FRβ (5' to 3') used in the RT-PCR reaction. "S" represents sense and "AS" represents antisense primers.

| OLIGO | Sense and Antisense Primers (5' to 3') |
|---|---|
| RFC-S | 5'-TTTCAGATTGCATCTTCTCTGTCT-3' (SEQ ID NO: 30) |
| RFC-AS | 5'-GAAGTAGATGATGGACAGGATCAG-3' (SEQ ID NO: 31) |
| FRα-S | 5'-TTCTAGTGTGGGTGGCTGTAGTAG-3' (SEQ ID NO: 32) |
| FRα-AS | 5'-CACAGTGGTTCCAGTTGAATCTAT-3' (SEQ ID NO: 33) |
| FRβ-S | 5'-CTTATGCAAAGAGGACTGTCAGC-3' (SEQ ID NO: 34) |
| FRβ-AS | 5'-CTGACCTTGTATGAGTGACTCCAG-3' (SEQ ID NO: 35) |

The product sizes were 189 bp for RFC, 234 bp for FRα and 201 bp for FRβ. PCR was employed using the Expand™ High Fidelity PCR system kit. Each reaction mixture contained 95 μl water, 20 μl 10x buffer, 40 μl enhancer, 4 μl dNTPs, 1 μl Taq polymerase and 20 μl cDNA. PCR amplification consisted of 40 cycles of 1 min denaturation at 94°C, 1.30 min annealing at 58°C and 2 min extension at 72°C. Subsequently, the amplified PCR products were analyzed by 2% agarose gel electrophoresis with ethidium bromide staining along with a DNA ladder (GeneRuler™). There was no apparent difference between the older and the younger passage, suggesting no loss of expression of the transporter genes by further splitting (Figure 5).

Isolation of Total Cellular RNA, Quantification of Isolated RNA and Reverse Transcriptase Real-Time PCR Reaction

For this purpose, Caco-2 cells (passage 54) were seeded on 24-well plate with a concentration of 100,000 cells/well. On the $16^{th}$ day, total RNA was extracted from Caco-2 cell monolayers using the RNeasy Mini Kit according to the instructions of the manufacturer.

Quantification of isolated RNA was based on spectrophotometric analysis. 3 μl of isolated RNA together with 97 μl of RNase-free water was read at 260 nm wavelength against RNase-free water that served as blank.

To perform the real-time PCR, a QuantiTect Probe RT-PCR Kit was used. The reactions were run in a real-time PCR instrument. The sequences of TaqMan probes, sense and antisense oligonucleotides are shown in Table 3 and Table 4, respectively.

**Table 3** The sequences of the TaqMan probes for RFC, FRα and FRβ used in RT real-time PCR reaction.

| Protein Name | Gene Symbol | TaqMan Probes (5' to 3') |
|---|---|---|
| RFC | SLC19A1 | 5' FAM-TCCGCAAGCAGTTCCAGTTATACTCCG (SEQ ID NO: 36)-TAMRA 3' |
| FRα | FOLR1 | 5' FAM-CATTTCTACTTCCCCACACCCACTGTT (SEQ ID NO: 37)-TAMRA 3' |
| FRβ | FOLR2 | 5' FAM-TTGTTAACTCCTGAGGTCCAGTCCCAT (SEQ ID NO: 38)-TAMRA 3' |

**Table 4** The sequences of sense and antisense primers for RFC, FRα and FRβ used in RT real-time PCR reaction.

| Oligo | Sense and Antisense Primers (5' to 3') |
|---|---|
| RFC-S | 5'-ACCATCATCACTTTCATTGTCTC-3' (SEQ ID NO: 39) |
| RFC-AS | 5'-ATGGACAGGATCAGGAAGTACA-3' (SEQ ID NO: 40) |
| FRα-S | 5'-ACTGGACTTCAGGGTTTAACAAG-3' (SEQ ID NO: 41) |
| FRα-AS | 5'-GTAGGAGTGAGTCCAGATTTCATT-3' (SEQ ID NO: 42) |
| FRβ-S | 5'-TATGCAAAGAGGACTGTCAGC-3' (SEQ ID NO: 43) |
| FRβ-AS | 5'-GGGAAGTAGGACTCAAAGGTG-3' (SEQ ID NO: 44) |
| GAPDH-S | 5'-AGCCTCAAGATCATCAGCAATG-3' (SEQ ID NO: 45) |
| GAPDH-AS | 5'-CACGATACCAAAGTTGTCATGGA-3' (SEQ ID NO: 46) |

Quantitative TaqMan PCR was performed in 96-well plates using a final volume of 25 μl. The components and volume of each component for the reaction were as shown in Table 5.

**Table 5** The components and volume of each component for Quantitative TaqMan PCR.

| Component | Volume [μl] |
|---|---|
| Sense primer [10 μmol/L] | 2 |
| Antisense primer [10 μmol/L] | 2 |
| Taqman probe [10 μmol/L] | 1 |
| RT-PCR Master Mix | 12.5 |
| QuantiTect Probe RT Mix | 0.25 |
| dNTPs | 0.5 |
| $MgCl_2$ | 1.75 |
| Template RNA [0.1 μg/μl] | 5 |
| TOTAL | 25 |

The reaction tubes were prepared as above and were placed in the real-time PCR instrument. The reaction was performed starting with a 30 min reverse transcription reaction at 50°C followed by the activation of Taq polymerase for 15 min at 95°C. 50 cycles of denaturation at 94°C for 15 s and combined primer annealing/extension at 60°C for 1 min were employed. The fluorescence increase of FAM was automatically measured during PCR.
For normalization of the gene levels, glyceraldehyde-3-phosphate dehydrogenase (GAPDH) was used to correct for minor variations in the input RNA amount or inefficiencies in the reverse transcription. The relative expression level of the target gene was normalized to the endogenous control (GAPDH) according to the equation below:

$$\Delta C_T = C_T(\text{target}) - C_T(\text{control})$$

where $C_T$ is the cycle number at the threshold and $\Delta C_T$ is the difference between the $C_T$ values of the target and the normaliser. SLC19A1 (RFC gene) was chosen as the reference for the comparison. The comparative $\Delta\Delta C_T$ is the difference between each sample's $\Delta C_T$ and the reference's $\Delta C_T$. Accordingly, the comparative expression level was calculated with the formula: $2^{-\Delta\Delta CT}$

The three genes analyzed are expressed at different levels (Figure 6). The Caco-2 cells express a high level of FRα, which is consistent with previous findings (L. Matherly and I.D. Goldman, 2003, Vitamins and Hormones, 66: 403; Lacey S.W. et al., 1989, J. Clin Invest. 84:715). Thus this cell line is well suited to act as a model to study the effect of targeting cancerous cells.

**Example 3: Cytotoxicity and Uptake Experiments**

Culturing and Preparation of Caco-2 for uptake studies:

Caco-2 cells (passage number 45) were cultured at 37°C in an atmosphere of 5% $CO_2$ and 90% relative humidity in 75 $cm^2$ cell culture flasks containing Dulbecco's modified Eagle's medium (DMEM) supplemented with 10% fetal bovine serum (FBS), 1% nonessential amino acids, 100 U/ml penicillin and 100 μg/ml streptomycin. The cells were routinely split and seeded into 6-well plates (Nunclon™ Multidishes, Life Technologies GmbH, Karlsruhe, Germany) with 800.000 cells/well. The medium was changed three times a week. The development of the monolayers was examined under the microscope until the 21st day. Then the monolayer cultures were used for uptake studies.

Cytotoxicity Assay:

Prior to analyzing the uptake of DNA block copolymer micelles, their biocompatibility was assessed. For the determination of the toxicity of the micelles, Caco-2 cells were seeded in 96-well plates at a concentration of 2500 cells/ well. *In vitro* cytotoxicity was determined based on a XTT *in vitro* toxicology assay kit following the procedure of the manufacturer (Sigma-Aldrich Chemie GmbH, Steinheim, Germany). On the 21st day post-seeding, the cell monolayers were washed once with HBSS containing 5 mmol/L HEPES adjusted to pH 7.4. Cells were incubated with DNA-b-PPO copolymer and their FA-functionalized derivatives at a DNA-b-PPO concentration of 325 μg/ml for 3 h at 37°C (in the case of Dox-loaded micelles, 24 h of incubation time was employed; see example 7). After the incubation period, the medium was removed, monolayers were washed once with the buffer solution and the reconstituted XTT was added into each well with a volume of 100 μl and incubated for 2 h at 37°C. Subsequently, absorbance was measured at a wavelength of 450 nm. A reference measurement was also taken at a wavelength of 690 nm and subtracted from the measurement at 450 nm. The cytotoxicity of folic acid conjugated nanoparticles were compared with the cells without any treatment (control) (Figure 7). More than 75 % of the cells treated with the differentrDNA-*b*-PPO copolymer and their FA-functionalized derivatives were viable. Thus, the nanoparticles themselves are non-toxic to Caco-2 cells.

Uptake experiment

Caco-2 cells with a passage of 57 were seeded on 6-well plates with a density of 800,000 cells/well. The medium in each well was changed every other day. On day 21, the medium was removed and monolayers washed two times with HBSS containing 5 mmol/L HEPES adjusted to pH 7.4.

For tracking purposes 4% of the nanoparticles were additionally labelled with a fluorescent dye: PPO-b-DNA micelles were hybridised with 3'-Alexa488-functionalized oligonucleotides encoding the complementary sequence of the DNA corona so that the dye was located in the interior. Incubation mixtures were prepared in pH 7.4 HBSS buffer at a DNA-b-PPO concentration of 325 μg/ml for Out 28, Out 11, Out 2, In 28, In 11 .and In 2. After incubating with 2 ml of micelle solutions for 3 h at 37°C on a rotating shaker at 50 rpm, incubation solutions were removed and the monolayers were washed five times with ice-cold HBSS (pH 7.4). Subsequently, cells in each well were lysed with 0.6 ml of 1.25 mmol/L NaOH, cell lysates were transferred into eppendorf tubes and shaken overnight at room temperature. The next day, lysates were centrifuged and the fluorescence content of the supernatant in each tube was measured (Excitation: 500 nm, Emission: 595 nm). Experiments were carried out in triplicates and the resulting data were expressed as % of control (without any targeting unit) (Figure 8A). This method offers the possibility to quantitatively compare the uptake of nanoparticles. As shown in Figure 8A, an increasing number of FA entities at the surface of the micelles strongly promoted internalization. With only 2 targeting units present the uptake into the cells was comparable to non-functionalized DNA block copolymer micelles. When the average number of targeting units was adjusted to 28, the uptake increased by a factor of 10 compared to the control. In contrast, when the targeting moieties pointed towards the interior of the micelles the uptake was comparable with bare DNA-*b*-PPO aggregates. From these experiments three important conclusions can be drawn. The uptake of DNA block copolymer micelles strongly depends on the number of targeting units at the rim. Furthermore, the higher the number of FA entities, the more efficiently the nanoparticles are internalized. Finally, when the targeting units are hidden inside the nanoparticles they cannot be "recognized" by the folate receptors, indicating that the micelles remain intact and do not dissociate into isolated block copolymers.

Confocal laser scanning microscopy (CLSM):

For the microscopy analysis, Caco-2 cells were seeded at a density of 20,000 cells/$cm^2$ on chamber slides (Lab-Tek® Chamber Slide System, Nunc, Germany). The cell monolayers were incubated with 325 μg/ml of the DNA-b-PPO labelled with Alexa488 for 3 h, washed 5 times with pH 7.4 HBSS and after the addition of 100 μl of buffer the monolayers were analyzed with confocal laser scanning microscopy (excitation 488nm). CLSM has proven to be a powerful tool for acquiring high resolution images, 3-D reconstructions and visualizations of internalization of nanoparticles. Figure 8B shows the CLSM image of Caco-2 cells after 3h incubation with DNA block copolymer micelles labelled with 28 targeting units at the surface that exhibited the most efficient uptake. 3-D slicing experiments showed that the nanoparticles were internalized homogenously and did not only adsorb on the membrane (Data not shown). No distinct patterns of subcellular staining were observed.

**Example 4:**

Cytotoxicity of Doxorubicin (Dox)-loaded DNA block copolymers

After the optimization of the targeting properties of the nanoparticles, the cytotoxicity of DNA block copolymer micelles loaded with the widely used anticancer drug Doxorubicin (Dox) was investigated. Dox is known to have side effects such as cardiotoxicity and myelosuppression, therefore targeted delivery is vital. The preparation of Dox-loaded micelles and the determination of loading content were carried out according to the literature (Shuai, X.T. et al., 2004, J. Controlled Release 98:415). The drug payload was 5.6 % of the nanoparticle by weight. The viability of Caco-2 cells after 24h incubation with Dox-loaded DNA block copolymer micelles was compared with several control experiments. The percentage of surviving cells was acquired using a XTT cell proliferation assay. Figure 9A shows a schematic overview of the nanoparticles loaded with Dox. Figure 9B shows that Caco-2 cells incubated with Dox-loaded micelles equipped with targeting units (on average 28 FA on the surface; column A in Figure 9B) had a viability of 24.1 ± 2.5 %. The controls consisted of Dox-loaded micelles in the presence of non-conjugated FA (Figure 9B; column B), Dox-loaded micelles in the absence of any targeting unit, (Figure 9B; column C) and folic acid-conjugated micelles in the absence of Dox, (Figure 9B; column D) with viabilities of 63.5 ± 7.9 %, 68.3 ± 7.1 % and 75.9 ± 8.2 %, respectively. The cell mortalities of the control experiments were significantly lower than when the Dox-loaded micelles were outfitted with FA units, which strongly indicates efficient drug delivery into the tumour cells by the DNA block copolymer micelles with the aid of targeting moieties and thus the significant cytotoxicity of these nanoparticles.

## Claims

1. A micelle comprising an amphiphilic block copolymer, said amphiphilic block copolymer consisting of

   (a) a hydrophobic polymer attached to the 5' end of a first nucleic acid molecule, wherein said first nucleic acid molecule is hybridized with a second nucleic acid molecule, wherein a targeting unit capable of selectively binding to a specific cell type and/or tissue is attached to the 5' end of said second nucleic acid molecule; and/or
   (b) a hydrophobic polymer attached to the 3' end of a first nucleic acid molecule, wherein said first nucleic acid molecule is hybridized with a second nucleic acid molecule, wherein a targeting unit capable of selectively binding to a specific cell type and/or tissue is attached to the 3' end of said second nucleic acid molecule.

2. The micelle of claim 1, further comprising

   (a) an amphiphilic block copolymer consisting of a hydrophobic polymer attached to the 3' or 5' end of a nucleic acid molecule;
   (b) an amphiphilic block copolymer consisting of a hydrophobic polymer attached to the 3' or 5' end of a first nucleic acid molecule, wherein said first nucleic acid molecule is hybridized with a second nucleic acid molecule, wherein a hydrophilic drug is covalently attached via a cleavable linker to said second nucleic acid;
   (c) an amphiphilic block copolymer consisting of a hydrophobic polymer attached to the 3' or 5' end of a first nucleic acid molecule, wherein said first nucleic acid molecule is hybridized with a second nucleic acid molecule, wherein a diagnostic agent is covalently attached to said second nucleic acid; and/or
   (d) an amphiphilic block copolymer consisting of a hydrophobic polymer attached to the 3' or 5' end of a first nucleic acid molecule, wherein said first nucleic acid molecule is hybridized with a second nucleic acid molecule, wherein a moiety capable of avoiding detection by the immune system is covalently attached to said second nucleic acid.

3. The micelle of claim 1 or 2, wherein said targeting unit is a ligand of a surface marker of said specific cell type and/or tissue.

4. The micelle according to any one of claims 1 to 3, wherein said ligand of a surface marker is selected from the group consisting of folate, transferrin, antiestrogens, estrogens, monoclonal antibody trastuzumab, neutravidin and saccharides.

5. The micelle of claim 3 or 4, wherein said surface marker is selected from the group of folate receptors, transferrin receptors, Epidermal Growth Factor receptors and cell-surface estrogen receptors.

6. The micelle of any one of claims 1 to 5, wherein said targeting unit is an antibody, antibody fragment or aptamer.

7. The micelle according to any one of claims 2 to 6, wherein said moiety capable of avoiding detection by the immune system is selected from polyethylene glycol, poloxamines and poloxamers.

8. The micelle according to any one of claims 1 to 7, wherein said specific cell type and/or tissue is associated with a disease.

9. The micelle according to claim 8, wherein said specific cell type and/or tissue is a tumor cell.

10. The micelle according to any one of claims 1 to 9, wherein said specific cell type and/or tissue is a human cell type and/or tissue.

11. The micelle according to any one of claims 2 to 10, wherein said hydrophilic drug is selected from the group consisting of topotecan, irinotecan, bleomycin, doxorubicin hydrochloride and mitomycin.

12. The micelle according to any one of claims 2 to 11, wherein said diagnostic agent is selected from the group consisting of folate-based radiodiagnostics, gallium-based radiodiagnostics, indium-based radiodiagnostics, technetium-based radiodiagnostics and near-infrared excitable fluorescent agents.

13. The micelle according to any one of claims 1 to 12, further comprising a hydrophobic drug.

**14.** The micelle of claim 13, wherein the hydrophobic drug is selected from the group consisting of Altretamine, Bexarotene, Methotrexate, Trimetrexate, Edatrexate, Piritrexim, Paclitaxel, Docetaxel, Tripentones, Doxorubicin, Bicalutamide and Cisplatin.

**15.** The micelle according to any one of claims 1 to 14, wherein the nucleic acid molecules are oligonucleotides or siRNAs.

**16.** The micelle according to any one of claims 1 to 15, wherein said hydrophobic polymer is selected from the group consisting of polypropylene oxide, poly(D,L-lactic-co-glycolic acid), polybutadiene and polyisoprene.

**17.** A composition comprising the micelle according to any one of claims 1 to 16.

**18.** The composition of claim 17 which is a pharmaceutical composition optionally further comprising a pharmaceutically acceptable carrier, excipient and/or diluent.

**19.** The composition of claim 17 which is a diagnostic composition.

**20.** A method of killing a specific target cell and/or tissue, the method comprising exposing the specific target cell and/or tissue to the micelle according to any one of claims 1 to 16 or to the composition of claim 17 or 18 wherein said targeting unit and/or said hydrophilic drug, if present, and/or said hydrophobic drug, if present, is/are (a) cytotoxic agent.

**21.** Use of the micelle according to any one of claims 1 to 16 for the preparation of a pharmaceutical composition for the treatment of cancer, neurodegenerative diseases, hepato-biliary diseases, cardiovascular diseases or pulmonary diseases.

**22.** Kit comprising the micelle according to any one of claims 1 to 16.

A)

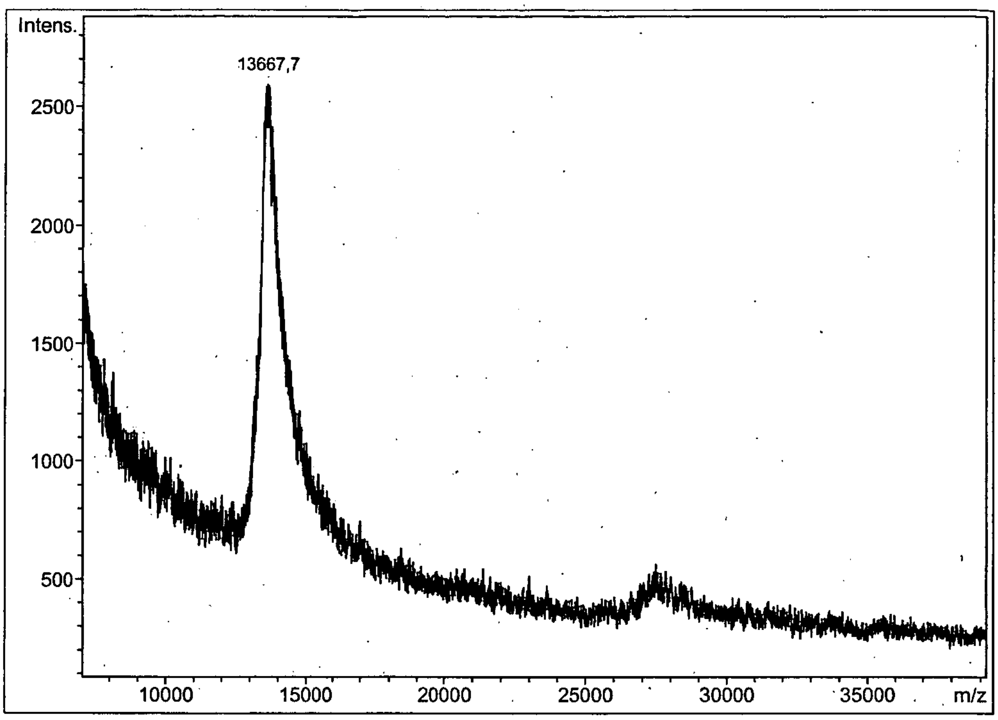
Intens.
13667,7
2500
2000
1500
1000
500
10000
15000
20000
25000
30000
35000
m/z

B)

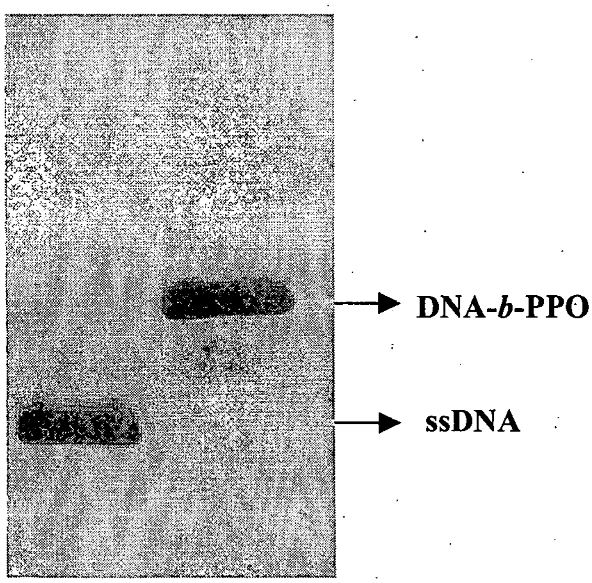
DNA-*b*-PPO
ssDNA

**Figure 1**

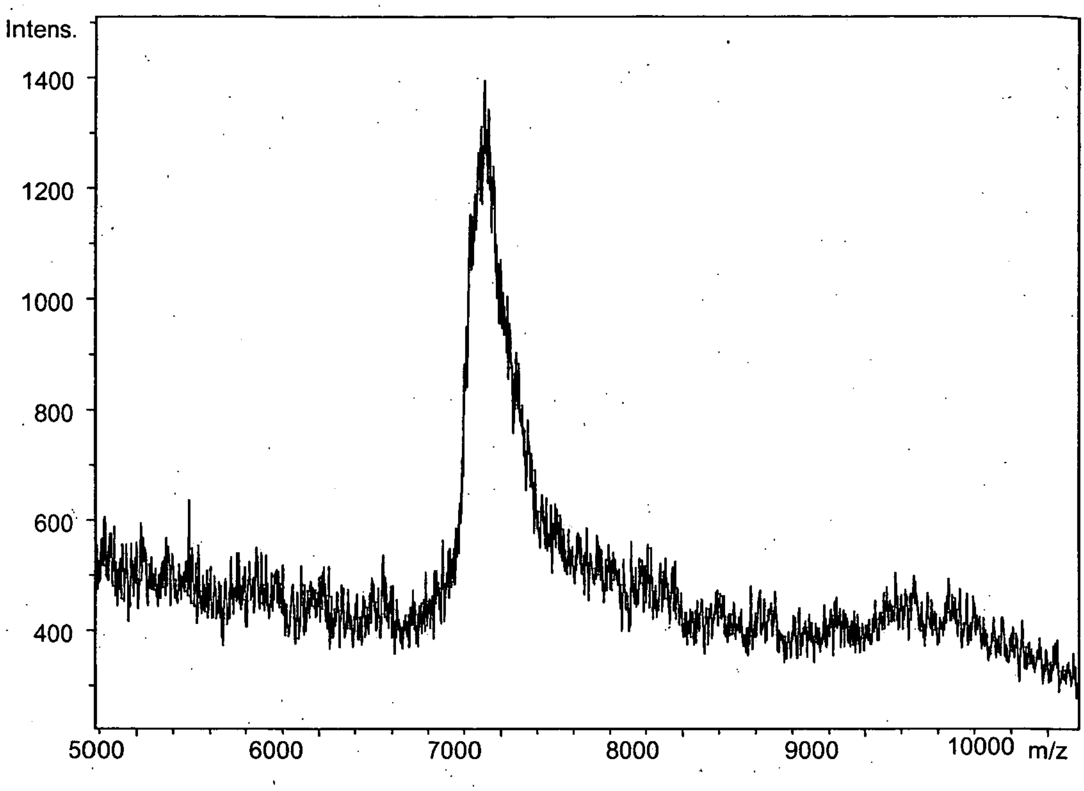


Figure 2

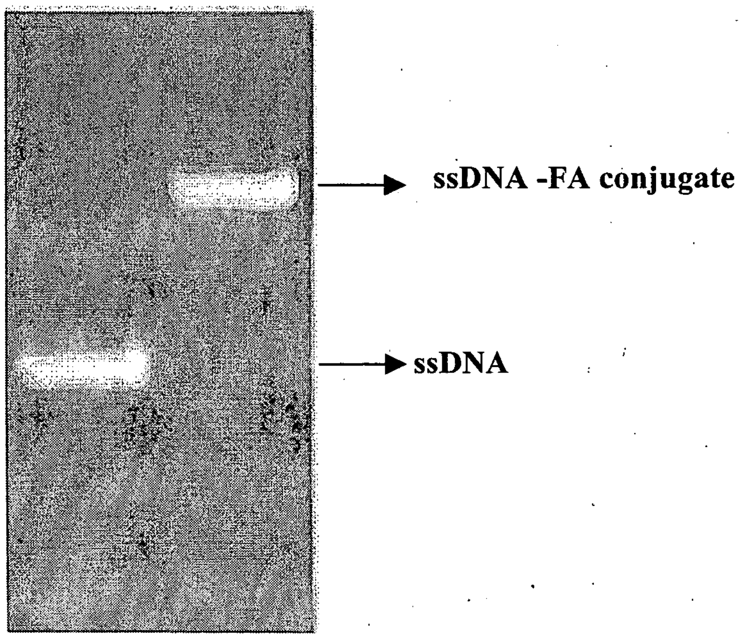


Figure 3

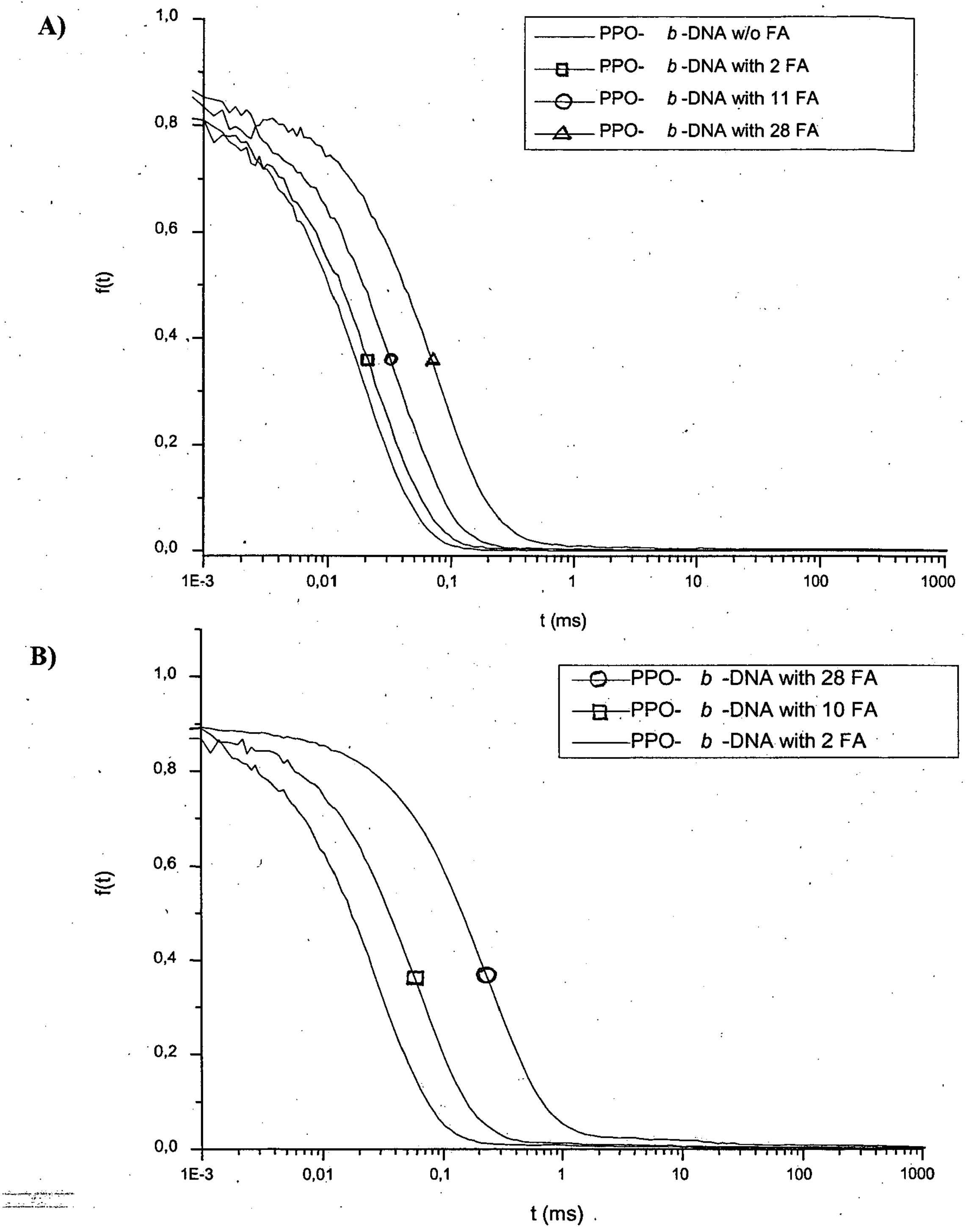
A)
1,0
0,8
0,6
0,4
0,2
0,0
f(t)
PPO- b -DNA w/o FA
PPO- b -DNA with 2 FA
PPO- b -DNA with 11 FA
PPO- b -DNA with 28 FA
1E-3
0,01
0,1
1
10
100
1000
t (ms)
B)
1,0
0,8
0,6
0,4
0,2
0,0
f(t)
PPO- b -DNA with 28 FA
PPO- b -DNA with 10 FA
PPO- b -DNA with 2 FA
1E-3
0,01
0,1
1
10
100
1000
t (ms)

**Figure 4**

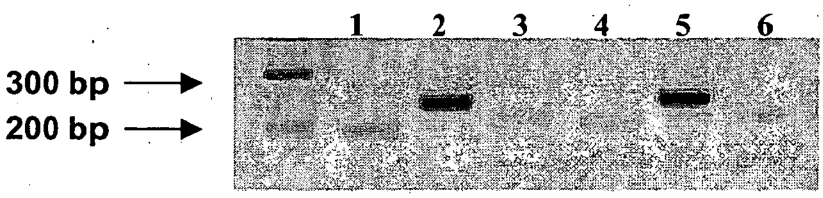


Figure 5

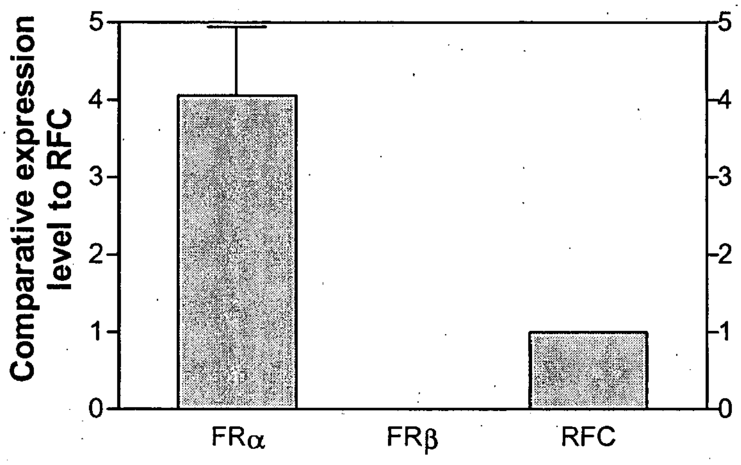


Figure 6

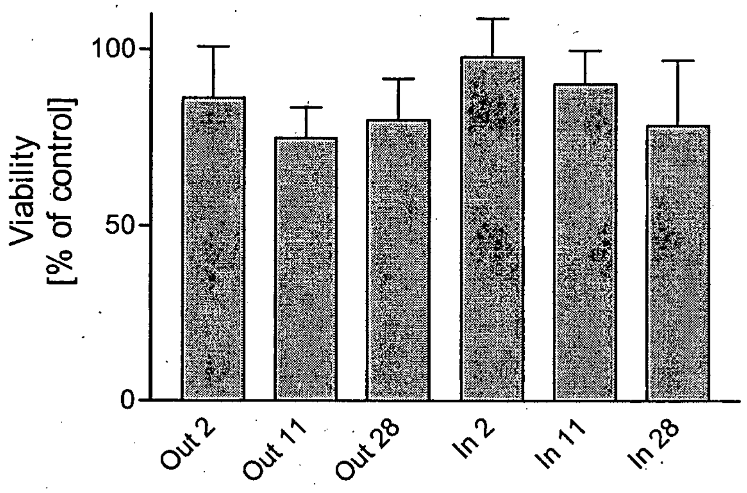


**Figure 7**

A)

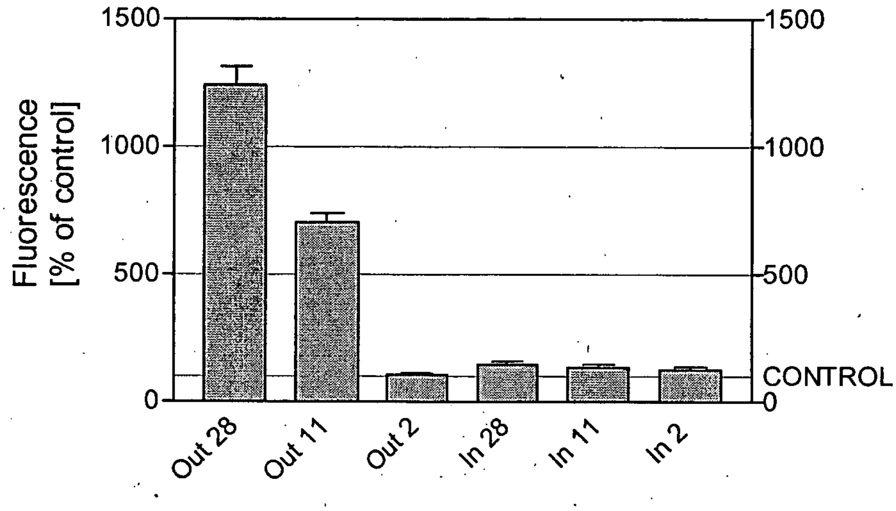
1500
1000
500
0
Fluorescence
[% of control]
1500
1000
500
0
CONTROL
Out 28
Out 11
Out 2
In 28
In 11
In 2

B)

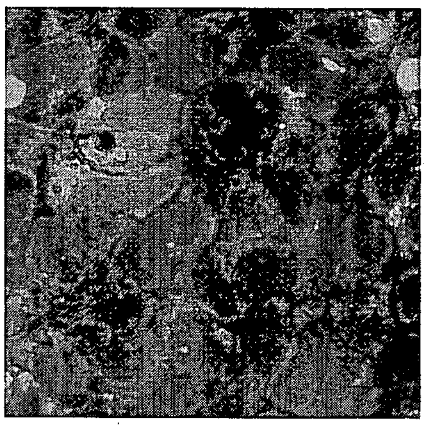

Figure 8

A)

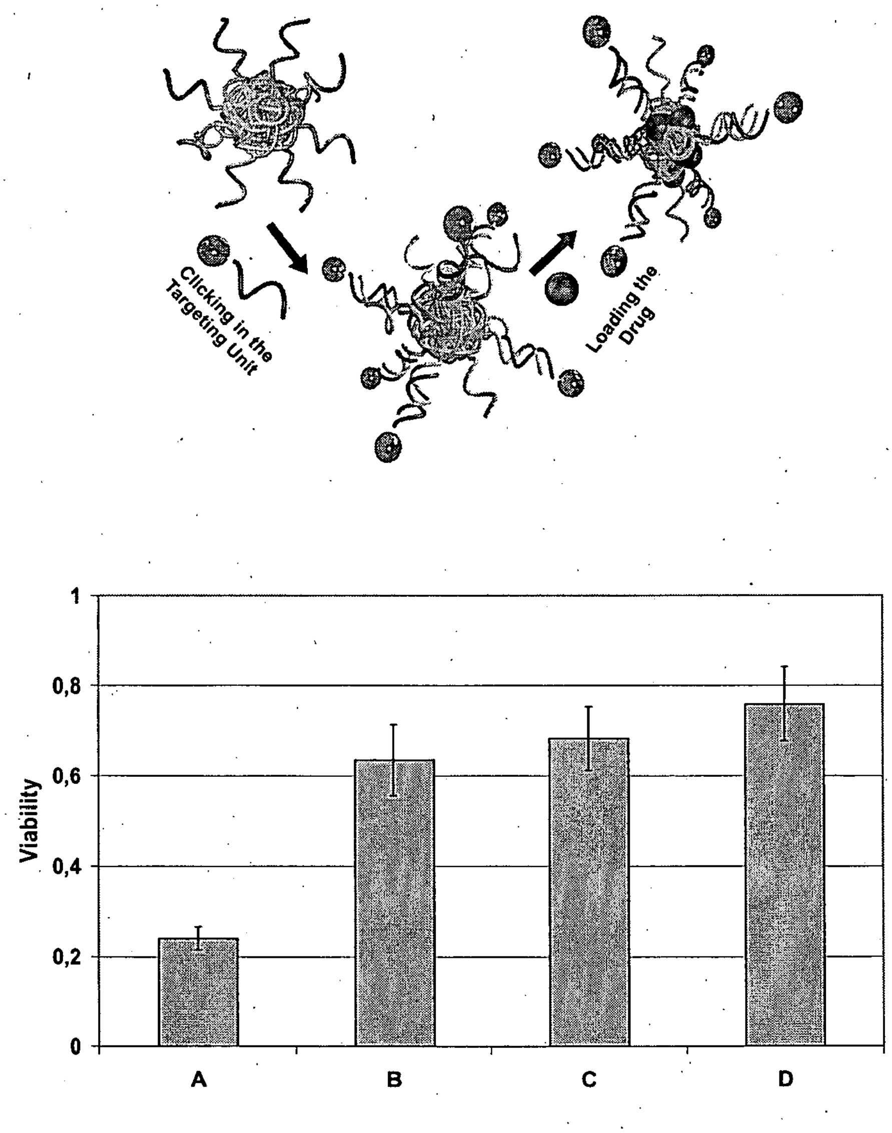
Clicking in the Targeting Unit
Loading the Drug
1
0,8
0,6
0,4
0,2
0
Viability
A
B
C
D

B)

Figure 9

European Patent Office

## PARTIAL EUROPEAN SEARCH REPORT

which under Rule 63 of the European Patent Convention shall be considered, for the purposes of subsequent proceedings, as the European search report

Application Number
EP 07 01 5900

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MANSOURI ET AL: "Characterization of folate-chitosan-DNA nanoparticles for gene therapy" BIOMATERIALS, vol. 27, no. 9, March 2006 (2006-03), pages 2060-2065, XP005204064 ISSN: 0142-9612 * abstract * * page 2064 * ----- | 1-22 | INV. A61K47/48 A61P35/00 |
| Y | DAUTY E ET AL: "Intracellular delivery of nanometric DNA particles via the folate receptor" BIOCONJUGATE CHEMISTRY, vol. 13, no. 4, July 2002 (2002-07), pages 831-839, XP002326611 ISSN: 1043-1802 * abstract * * figure 1 * ----- | 1-22 | |
| | | | TECHNICAL FIELDS SEARCHED (IPC) |
| Y | US 2004/141922 A1 (KLAVENESS JO [NO] ET AL) 22 July 2004 (2004-07-22) * examples 9,10 * ----- -/-- | 1-22 | A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| The Hague | 7 February 2008 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

European Patent Office

# PARTIAL EUROPEAN SEARCH REPORT

Application Number
EP 07 01 5900

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| Y | WANG S ET AL: "Folate-mediated targeting of antineoplastic drugs, imaging agents, and nucleic acids to cancer cells" JOURNAL OF CONTROLLED RELEASE, vol. 53, no. 1-3, 30 April 1998 (1998-04-30), pages 39-48, XP004121255 ISSN: 0168-3659 * abstract * * page 44, right-hand column, paragraph 9. - page 47, left-hand column * ----- | 1-22 | |
| Y | HATTORI Y ET AL: "Enhanced in vitro DNA transfection efficiency by novel folate-linked nanoparticles in human prostate cancer and oral cancer" JOURNAL OF CONTROLLED RELEASE, vol. 97, no. 1, 31 May 2004 (2004-05-31), pages 173-183, XP004508985 ISSN: 0168-3659 * abstract * * page 177, paragraph RESULTS - page 180 * ----- | 1-22 | TECHNICAL FIELDS SEARCHED (IPC) |
| Y | MISLICK K A ET AL: "TRANSFECTION OF FOLATE-POLYSINE DNA COMPLEXES: EVIDENCE FOR LYSOSOMAL DELIVERY" BIOCONJUGATE CHEMISTRY, vol. 6, no. 5, 1 September 1995 (1995-09-01), pages 512-515, XP000542461 ISSN: 1043-1802 * abstract * * figures 1-5 * * page 514 - page 515 * ----- | 1-22 | |
| | -/-- | | |

1

EPO FORM 1503 03.82 (P04C10)

European Patent Office

# PARTIAL EUROPEAN SEARCH REPORT

Application Number
EP 07 01 5900

| Category | Citation of document with indication, where appropriate, of relevant passages | Relevant to claim | CLASSIFICATION OF THE APPLICATION (IPC) |
|---|---|---|---|
| Y | ALEMDAROGLU F E ET AL: "DNA multiblock copolymers" CHEMICAL COMMUNICATIONS, [Online] no. 13, 17 January 2007 (2007-01-17), pages 1358-1359, XP002468092 ISSN: 1359-7345 Retrieved from the Internet: URL:http://dx.doi.org/10.1039/b615276g> [retrieved on 2007-11-07] * the whole document * ----- | 1-22 | |
| Y | DING KE ET AL: "Engineering the structural properties of DNA block copolymer micelles by molecular recognition." ANGEWANDTE CHEMIE (INTERNATIONAL ED.), [Online] vol. 46, no. 7, 9 January 2007 (2007-01-09), pages 1172-1175, XP002468093 ISSN: 1433-7851 Retrieved from the Internet: URL:http://dx.doi.org/10.1002/anie.200603064> [retrieved on 2007-11-07] * figures * * page 1173 - page 1174 * ----- | 1-22 | TECHNICAL FIELDS SEARCHED (IPC) |
| | -/-- | | |

1

EPO FORM 1503 03.82 (P04C10)

European Patent Office

# PARTIAL EUROPEAN SEARCH REPORT

Application Number
EP 07 01 5900

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| Y | SAFAK, MERYEM ET AL: "Polymerase chain reaction as an efficient tool for the preparation of block copolymers" ADVANCED MATERIALS, [Online] vol. 19, no. 11, 4 May 2007 (2007-05-04), XP002468094 ISSN: 0935-9648 Retrieved from the Internet: URL:http://dx.doi.org/10.1002/adma.200700240> [retrieved on 2007-11-13] * figure 1 * * table 1 * * page 1504, left-hand column, paragraph EXPERIMENTAL * | 1-22 | |
| | ----- | | TECHNICAL FIELDS SEARCHED (IPC) |
| Y | ALEMDAROGLU FIKRI E ET AL: "DNA meets synthetic polymers--highly versatile hybrid materials." ORGANIC & BIOMOLECULAR CHEMISTRY, [Online] vol. 5, no. 9, 7 May 2007 (2007-05-07), pages 1311-1320, XP002468095 ISSN: 1477-0520 Retrieved from the Internet: URL:http://dx.doi.org/10.1039/b617941j> [retrieved on 2007-11-07] * abstract * * page 1314 * * page 1319, left-hand column * | 1-22 | |
| | ----- | | |
| | -/-- | | |

1

EPO FORM 1503 03.82 (P04C10)

European Patent Office

# PARTIAL EUROPEAN SEARCH REPORT

Application Number
EP 07 01 5900

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | ALEMDAROGLU FIKRI E ET AL: "DNA-templated synthesis in three dimensions: Introducing a micellar scaffold for organic reactions." ANGEWANDTE CHEMIE (INTERNATIONAL ED.), [Online] vol. 45, no. 25, 19 June 2006 (2006-06-19), pages 4206-4210, XP002468096 ISSN: 1433-7851 Retrieved from the Internet: URL:http://dx.doi.org/10.1002/anie.200600524> [retrieved on 2007-11-07] * schemes * * figures * ----- | 1-22 | |
| | | | TECHNICAL FIELDS SEARCHED (IPC) |

1

EPO FORM 1503 03.82 (P04C10)

European Patent Office

INCOMPLETE SEARCH
SHEET C

Application Number
EP 07 01 5900

Although claim 20 is directed to a method of treatment of the human/animal body (Article 53(c) EPC), a search has been carried out, based on the alleged effects of the compound/composition.

-----

# ANNEX TO THE EUROPEAN SEARCH REPORT ON EUROPEAN PATENT APPLICATION NO. EP 07 01 5900

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2008

| Patent document cited in search report | Publication date | Patent family member(s) | Publication date |
|---|---|---|---|
| US 2004141922 A1 | 22-07-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4946778 A
- US 4904479 A


### Non-patent literature cited in the description


- **LEAMON, CP ; REDDY, JA.** *Adv. Drug Delivery Rev.,* 2004, vol. 56, 1127
- **QUINTANA, A. et al.** *Pharm Res,* 2002, vol. 19, 1310
- **PAN, D. et al.** *Chem. Commun.,* 2003, 2400
- **YOO, HS ; PARK, TG.** *J. Controlled Release,* 2004, vol. 96, 273
- **PARK, EK et al.** *J. Controlled Release,* 2005, vol. 109, 158
- **DAS, M et al.** *Adv. Mater.,* 2006, vol. 18, 80
- **ALEMDAROGLU, FE ; HERRMANN, A.** *Org. Biomol. Chem.,* 2007
- **ALEMDAROGLU, FE et al.** *Chem. Commun.,* 2007, 1358
- **DING, K et al.** *Angew. Chem., Int. Ed.,* 2007, vol. 46, 1172
- **JEONG, JH ; PARK, TG.** *Bioconjugate Chem.,* 2001, vol. 12, 917
- **LI, Z et al.** *Nano Lett,* 2004, vol. 4, 1055
- **ALEMDAROGLU, FE et al.** *Angew. Chem., Int. Ed.,* 2006, vol. 45, 4206
- **BRAASCH ; COREY.** *Chem Biol,* 2001, vol. 8, 1
- **NIELSEN et al.** *Science,* 1991, vol. 254, 1497
- **EGHOLM et al.** *Nature,* 1993, vol. 365, 666
- **DEMIDOV et al.** *Biochem. Pharmacol.,* 1994, vol. 48, 1310-1313
- **WITTUNG et al.** *Nature,* 1994, vol. 368, 561-563
- **RAY ; NORDEN.** *Faseb J.,* 2000, vol. 14, 1041-1060
- **ZAMORE.** *Nat Struct Biol,* 2001, vol. 8 (9), 746-50
- **TUSCHL T.** *CHEMBIOCHEM.,* 2001, vol. 2, 239-245
- **SCHERR ; EDER.** *Cell Cycle,* February 2007, vol. 6 (4), 444-9
- **LEUNG ; WHITTAKER.** *Pharmacol Ther.,* August 2005, vol. 107 (2), 222-39
- **DE FOUGEROLLES et al.** *Nat. Rev. Drug Discov.,* 2007, vol. 6, 443-453
- **ZHANG et al.** *Curr Top Med Chem.,* 2006, vol. 6 (9), 893-900
- **MANOHARAN.** *Curr Opin Chem Biol.,* December 2004, vol. 8 (6), 570-9
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press, 1985
- *J. Controlled Release,* 1997, vol. 43, 197-212
- **M. OISHI et al.** *Chembiochem,* 2005, vol. 6, 718-725
- **M. OISHI et al.** *Biomacromolecules,* 2003, vol. 4, 1426-1432
- **J. H. JEONG et al.** *Bioconjugate Chemistry,* 2003, vol. 14, 473-479
- **L. MATHERLY ; I.D. GOLDMAN.** Vitamins and Hormones. Academic Press, 2003, vol. 55, 403
- *Advanced Materials,* vol. 18 (1), 80-83
- **FANG et al.** *Chem Res Toxicol,* 2001, vol. 14 (3), 280-294
- **WILEY et al.** *Trends Cell Biol,* 2003, vol. 13, 43-50
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **KÖHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **SELA.** *Science,* 1969, vol. 166, 1365
- **LAVER.** *Cell,* 1990, vol. 61, 553-6
- **ELLINGTON et al.** *Nature,* 1990, vol. 346 (6287), 818-822

- **BOCK et al.** *Nature,* 1992, vol. 355 (6360), 564-566
- **COHEN et al.** *PNAS,* 1998, vol. 95 (24), 14272-14277
- **SHUAI et al.** *J. Controlled Release,* 2004, vol. 98, 415
- **O'CONNOR R.** *Anticancer Res.,* 2007, vol. 27, 1267-72
- **P. ANDERLE et al.** *J. Pharm. Sci.,* 1998, vol. 87, 757-762
- **S. DÖPPENSCHMITT et al.** *Pharm. Res.,* 1998, vol. 15, 1001-1006
- *Journal of Experimental Medicine,* 2005, vol. 202, 1575-1585
- *Science,* 2004, vol. 303, 1526-1529
- **MILLER, D.W. et al.** *Bioconjugate Chem.,* 1997, vol. 8, 649
- **ALEMDAROGLU, F. E. et al.** *Angew. Chem., Int. Ed.,* 2006, vol. 45, 4206
- **L. MATHERLY ; I.D. GOLDMAN.** *Vitamins and Hormones,* 2003, vol. 66, 403
- **LACEY S.W. et al.** *J. Clin Invest.,* 1989, vol. 84, 715
- **SHUAI, X.T. et al.** *J. Controlled Release,* 2004, vol. 98, 415